# EUROPEAN PATENT APPLICATION

(11) **EP 2 246 429 A1**
(43) Date of publication of application: **03.11.2010**
(21) Application number: 09709975.8
(22) Date of filing: 10.02.2009
(51) Int. Cl.: C12N 15/09, A01K 67/02, A01K 67/027, C12Q 1/68

(54) **NON-HUMAN MAMMAL MODEL OF EPILEPSY**

(30) Priority: 12.02.2008 JP 2008031002
(71) Applicant: Fukuoka University, Junan-ku, Fukuoka-shi Fukuoka 814-0180 (JP); Hirosaki University, Hirosaki-shi, Aomori 036-8560 (JP)
(72) Inventor: HIROSE, Shinichi, Fukuoka-shi Fukuoka 814-0180 (JP); KANEKO, Sunao, Hirosaki-shi Aomori 036-8560 (JP)
(74) Representative: Rigby, Barbara Nicole
(86) International application number: PCT/JP2009/052230
(87) International publication number: WO 2009/101939

(57) **Abstract**

The present invention provides a genuine epilepsy model animal as an improvement over conventional epilepsy model animals which are socalled seizures model animals mainly causing seizure attacks to be forcibly induced. Also provided is a method that allows for easy identification of the recombinants.

The model non-human mammalian animal for human epilepsy according to the present invention is a human epilepsy model non-human mammalian animal, such as a rat model, that has the same genetic defects as those in human epilepsy. The model non-human mammalian animal has a mutated gene obtained by introducing a genetic mutation to the non-human mammalian DNA of the α4 subunit (*CHRNA*4) or β2 subunit (*CHRNB*2) of the neuronal nicotinic acetylcholinergic receptor gene associated with human autosomal dominant nocturnal frontal lobe epilepsy, and by introducing a specific probe. The human epilepsy model non-human mammalian animal of the present invention allows for easy identification of the recombinants.

## Description

### TECHNICAL FIELD

The present invention relates to a non-human mammal model of epilepsy. More particularly, the present invention relates to a model non-human mammalian animal for human epilepsy that has genetic defects homologous to the genes associated with human autosomal dominant nocturnal frontal lobe epilepsy and that spontaneously develops epileptic seizures. The present invention also relates to mutated genes that allow for easy identification of genetically recombinant individuals, a method of construction thereof, and a method for identifying such genes.

### BACKGROUND ART

Epilepsy is a relatively common neurological disorder affecting about 2% of the population in Japan. However, the molecular cause of the disease has remained unclarified for many years because epilepsy is the collective term including many different forms of the disease. In recent years, genetic defects regarding epilepsy are gradually becoming clarified with the focus placed mostly on familial epilepsy.

Autosomal dominant nocturnal frontal lobe epilepsy as one form of familial epilepsy is characterized by nocturnal epileptic seizures. There is the report that this is caused by genetic mutations in the α4 and β2 subunit genes *CHRNA4* and *CHRNB2* associated with the neuronal acetylcholinergic receptor (Non-Patent Document 1). Three kinds of genetic mutations S284L, S280F, and 291-292insL have so far been reported in CHRNA4 (Non-Patent Documents 2, 3, 4, and 5). In *CHRNB2*, three kinds of genetic mutations V287L, V287M, and I312M have so far been reported (Non-Patent Documents 6, 7, and 8).

As a means for the development and advancement of the diagnostics and treatment of epilepsy, socalled "epilepsy model animals" have been used. Conventional epilepsy model animals are "seizure model animals" which make use of an electrical stimulus or a convulsant such as pentylenetetrazol. Transgenic non-human mammals which develop epilepsy-like seizures in response to the introduction of a sugar-binding antibody gene are disclosed (Patent Document 1). Non-human animals which lack a function of the µ3B gene on the chromosome and develop a tonic-clonic seizure or symptoms of epilepsy in response to a postural change are created (Patent Document 2). These conventional model animals can be used as model animals of seizure attacks, however they are not called genuine model animals for human epilepsy from the standpoint of molecular biology.

Previously, the inventors of the present invention have found that human autosomal dominant nocturnal frontal lobe epilepsy is caused by the substitution of Ser for Leu at position 284 of the α4 subunit (CHRNA4) of the neuronal nicotinic acetylcholinergic receptor gene (Non-Patent Document 9).

Based on this finding, the inventors of the present invention have created a genetically recombinant epilepsy model animal with a mutation introduced into the rat neuronal acetylcholinergic receptor gene *CHRNA4* by genetic modifications (Patent Document 3).

The prior art techniques, however, require screening of large numbers of recombinants, because of the low probability of homologous recombination occurring in creating the genetically recombinant model animals. Further, the screening of recombinants requires sequencing of a part of the gene, adding time and cost to the procedure. Under these circumstances, demands have been made for the development of a method for distinguishing recombinants without sequencing the gene in order to save time and cost greatly.
[Non-Patent Document 1] Hirose, S., et al., Neurology 53:1749-1753, 1999
[Non-Patent Document 2] Steinlein, O.K., et al. A missense mutation in the neuronal nicotinic acetylcholine receptor alpha 4 subunit is associated with autosomal dominant nocturnal frontal lobe epilepsy. Nat Genet 11, 201-203 (1995).
[Non-Patent Document 3] Hirose, S., et al. A novel mutation of CHRNA4 responsible for autosomal dominant nocturnal frontal lobe epilepsy. Neurology 53, 1749-1753 (1999).
[Non-Patent Document 4] Steinlein, O.K., et al. Independent occurrence of the CHRNA4 Ser248Phe mutation in a Norwegian family with nocturnal frontal lobe epilepsy. Epilepsia 41, 529-535 (2000).
[Non-Patent Document 5] Steinlein, O.K., et al. An insertion mutation of the CHRNA4 gene in a family with autosomal dominant nocturnal frontal lobe epilepsy. Hum Mol Genet 6, 943-947 (1997).
[Non-Patent Document 6] De Fusco, M., et al. The nicotinic receptor b2 subunit is mutant in nocturnal frontal lobe epilepsy. Nat Genet 26, 275-276 (2000).
[Non-Patent Document 7] Phillips, H.A., et al. CHRNB2 is the second acetylcholine receptor subunit associated with autosomal dominant nocturnal frontal lobe epilepsy. Am J Hum Genet 68, 225-231 (2001).
[Non-Patent Document 8] Bertrand, D., et al. The CHRNB2 mutation I312M is associated with epilepsy and distinct memory deficits. Neurobiol Dis 20, 799-804 (2005).
[Non-Patent Document 9] Hirose, S., et al. A novel mutation of CHRNA4 responsible for autosomal dominant nocturnal frontal lobe epilepsy. Neurology 53, 1749-1753 (1999).
[Patent Document 1] JP-A-2006-141217
[Patent Document 2] Japanese Patent No. 3853136
[Patent Document 3] JP-A-2005-245361

### DISCLOSURE OF INVENTION

### Problems to be solved by the Invention

In order to meet such demands, the inventors of the present invention have conducted extensive studies, and found that a model animal for epilepsy can be obtained from a non-human mammalian animal by introducing genetic mutations associated with the genetic defects in human *CHRNA4* or *CHRNB2* into the cDNA of the homologous gene *Chrna*4 or *Chrnb*2 of a non-human animal, respectively, and by introducing a probe to a part of the base sequence of the mutated cDNA, a probe being devised to have a base sequence that differs from this part of the base sequence, but codes for the same amino acid sequence. The inventors have also found that the non-human mammalian animal created this way allows an easy identification of the recombinant individuals, and enables the mRNA expressed from the recombinant gene to be easily detected by distinguishing it from the mRNA of the original homologous gene. It has further been found that the recombinant individuals created by introducing genetic mutations associated with the genetic defects in human *CHRNA4* or *CHRNB2* into the cDNA of the homologous gene *Chrna*4 or *Chrnb*2 of the non-human animal can easily be identified by devising the mutation introducing site and the mutation in such a manner that a new restriction site of a restriction enzyme can be created upon introduction of the mutation. The present invention was completed based on these findings.

It is accordingly an object of the present invention to provide a model non-human mammalian animal for human epilepsy, such as a model rat, which has the same genetic defects as those in human epilepsy. The model non-human mammalian animal includes a mutated gene obtained by introducing a genetic mutation into the non-human mammalian DNA of the α4 subunit *(CHRNA4)* or β2 subunit (*CHRNB2*) of the neuronal nicotinic acetylcholinergic receptor gene associated with human autosomal dominant nocturnal frontal lobe epilepsy, and by introducing a specific probe. The invention also has an object to provide a method for creating such model non-human mammalian animals for human epilepsy.

Another object of the present invention is to provide the mutated gene that includes the genetic mutation in the cDNA, and in which a part of the base sequence of the cDNA is replaced by a probe that has a base sequence different from this part of the base sequence, but codes for the same amino acid sequence. The invention also has an object to provide a method for creating such mutated genes.

In a preferred aspect, the present invention has an object to provide the mutated gene in which a new restriction site for a restriction enzyme is created by the introduction of the genetic mutation in the cDNA and to provide a method for introducing such mutations.

It is yet another object of the present invention to provide a method for the identification of a recombinant, which allows the recombinant individual to be easily identified with the restriction enzyme to be used for the restriction site of the restriction enzyme or with the restriction enzyme used to create the probe.

### Means for Solving the Problems

In order to achieve the foregoing objects, the present invention provides a model non-human mammalian animal for human epilepsy, wherein a mutated gene is created by introducing a genetic mutation to the gene *Chrna*4 or *Chrnb*2 of a non-human mammalian animal homologous to the α4 subunit (*CHRNA*4) or β2 subunit (*CHRNB2*) of the neuronal nicotinic acetylcholinergic receptor gene associated with human autosomal dominant nocturnal frontal lobe epilepsy, respectively, and wherein a part of the base sequence is replaced by introducing a probe devised to have a base sequence that differs from this part of the base sequence, but codes for the same amino acid sequence.

In a preferred aspect, the present invention provides a model non-human mammalian animal for human epilepsy, wherein a new restriction site for a restriction enzyme is caused to appear by the introduction of the mutation.

Another object of the present invention is to provide a method for creating the model non-human mammalian animal for human epilepsy. The method comprises preparing a mutated gene by introducing a genetic mutation into the cDNA of the gene *Chrna*4 or *Chrnb*2 of a non-human mammalian animal homologous to the α4 subunit *CHRNA*4 or β2 subunit *CHRNB2* of the neuronal nicotinic acetylcholinergic receptor gene associated with human autosomal dominant nocturnal frontal lobe epilepsy, respectively, and by replacing a part of the base sequence of the cDNA by a probe that has a base sequence different from this part of the base sequence, yet codes for the same amino sequence, transferring the mutated gene to an expression vector, injecting the isolated DNA obtained by cutting it with an restriction enzyme into a fertilized egg, and transplanting the fertilized egg into a recipient female so as to create a recombinant.

The present invention also provides the mutated gene and a method for the production of the mutated gene, which includes one or more mutations introduced to its base sequence and in which a part of the cDNA base sequence is replaced by a probe that has a base sequence different from this part of the base sequence yet codes for the same amino sequence. In a preferred aspect of the invention, there is provided the mutated gene including a new restriction site for a restriction enzyme created by the introduction of the mutation, and a method for creating such mutated genes.

The present invention also provides a recombinant identification method which identifies the presence or absence of homologous recombination between the foreign gene as the mutated gene and the original gene of the host model non-human mammalian animal for human epilepsy, using the probe or the restriction enzyme for the restriction site.

### Advantage of the Invention

The model non-human mammalian animal for human epilepsy according to the present invention is highly advantageous in that it can be used as a genuine model animal for human epilepsy which naturally develops epileptic seizures, rather than a "seizure model animal" in which seizure attacks are forcibly induced with the use of an external stimulus or convulsants.

More specifically, the model non-human mammalian animal for human epilepsy of the present invention has the same genetic defects as those in human epilepsy so that the model non-human mammalian animal can be advantageously used as a genuine model animal for human epilepsy which naturally develops epileptic seizures as might occur in humans.

Another advantage of the model non-human mammalian animal for human epilepsy according to the present invention resides in an easy identification of a recombinant individual without sequencing the recombinants by the use of the restriction enzyme for the restriction site created by the introduction of the mutation or the use of the restriction enzyme for the probe introduced into the mutated gene. A further advantage of the present invention is to readily distinguish the mRNA expressing from the recombinant gene by PCR, *in situ* hybridization, etc. from the mRNA of the original *Chrna*4 or *Chrnb*2 of, for example, a rat.

### Brief Description of Drawings

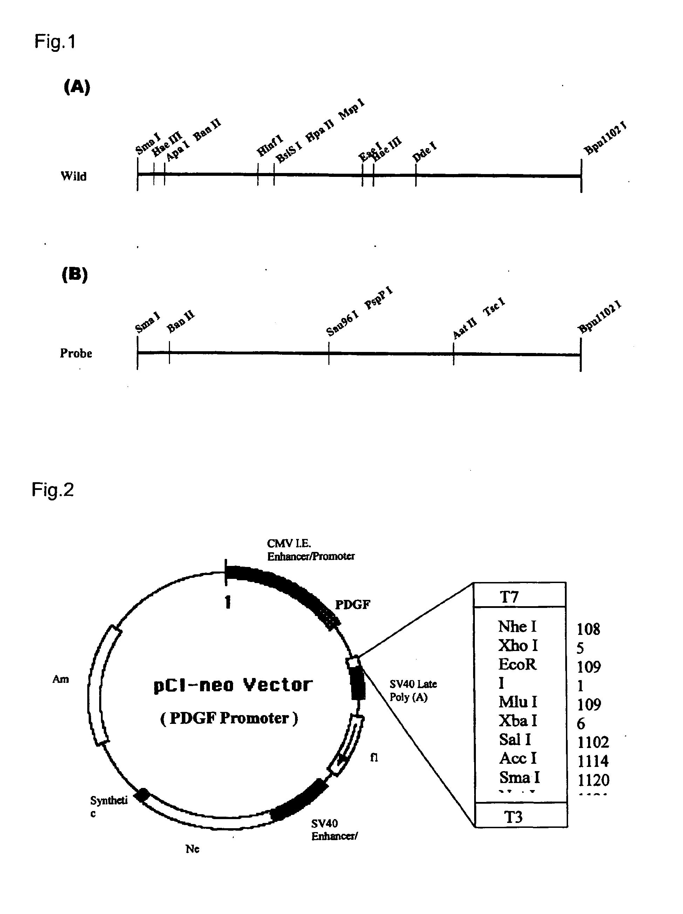

Fig. 1 is a diagram illustrating enzyme sites at the *Sma I*/*Bpu1102 I* site of *CHRNA4,* in which (A) shows the enzyme sites of wild-type *CHRNA4,* and (B) shows the enzyme sites of a probe.
Fig. 2 is a schematic diagram illustrating an expression vector constructed in Example 1.

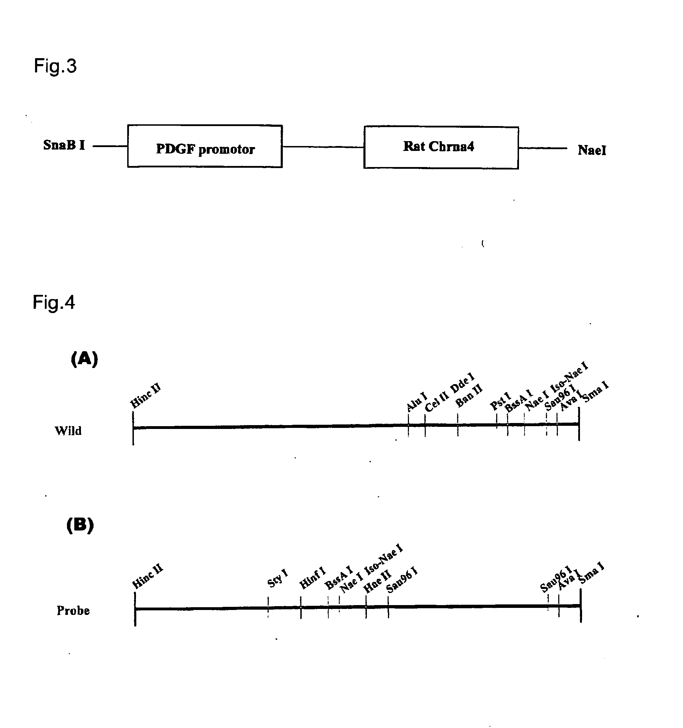

Fig. 3 is a schematic diagram showing a structure of a PDGF promoter and rat *Chrna*4 at *SnaB I*/*NaeI* site.
Fig. 4 is a diagram illustrating enzyme sites at the *Hinc II*/*Sma I* site of *CHRNB*2, in which (A) shows the enzyme sites of wild-type *CHRNB2,* and (B) shows the enzyme sites of a probe.
Fig. 5 is a schematic diagram illustrating an expression vector constructed in Example 4.
Fig. 6 is a schematic diagram showing a structure of a PDGF promoter and rat *Chrnb*2 at SnaB I/Dra III site.
Fig. 7 is a diagram representing the result of electroencephalography (EEG) for a transgenic rat (*Chrnb*2 V287L).

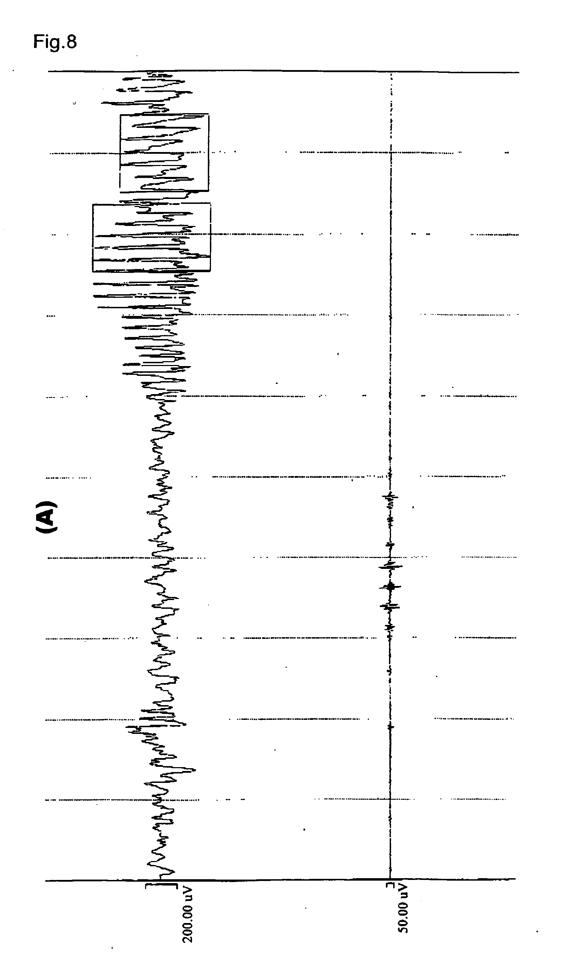

Fig. 8 is a magnified view of the portion A in Fig. 6.

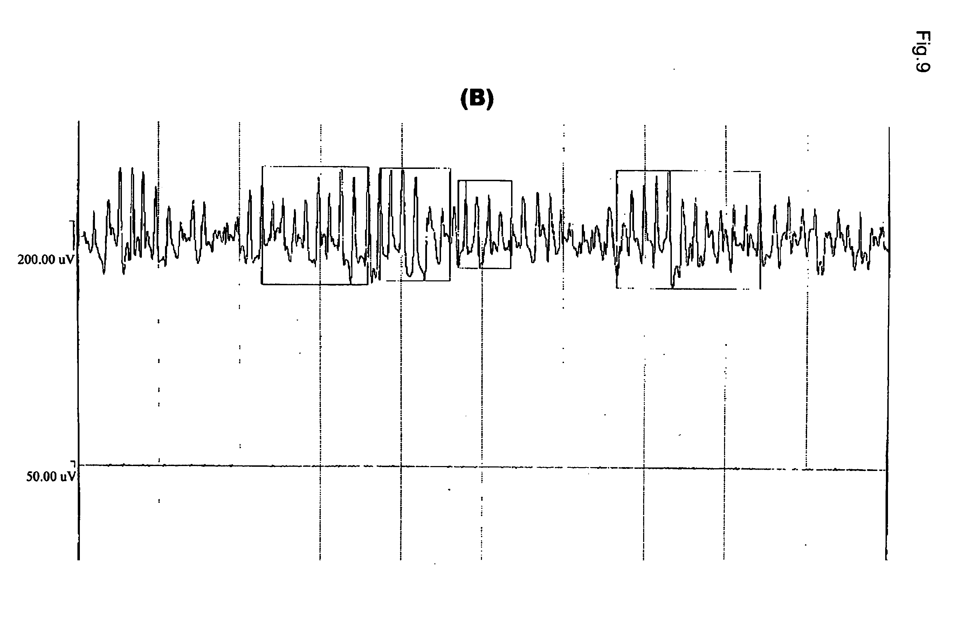

Fig. 9 is a magnified view of the portion B in Fig. 6.

### Best Mode for Carrying Out the Invention

A model non-human mammalian animal for human epilepsy according to the present invention is a genetically recombinant model non-human mammalian animal for human epilepsy, in which a genetic mutation of the α4 subunit (CHRNA4) or the β2 subunit (*CHRNB2*) of a neuronal nicotinic acetylcholinergic receptor gene associated with human autosomal dominant nocturnal frontal lobe epilepsy that is one form of familial epilepsy and that is characterized by an occurrence of nocturnal epileptic seizures. Moreover, the genetically recombinant model non-human mammalian animal for human epilepsy possesses a mutated gene into which a probe is introduced, a probe being designed to allow a portion of the base sequence of the gene to have a base sequence that differs from the base sequence of the original gene yet codes for the amino acid sequence identical thereto. In addition, the function of the α4 subunit (CHRNA4) or the β2 subunit (*CHRNB2*) of the neuronal nicotinic acetylcholinergic receptor gene is deleted or lacks in the model animal.

As employed herein, the terms "non-human mammalian animals" refer to mammalian animals other than human beings, including mouse, rat, rabbit, dog, cat, swine, cattle, horse and so on. Among them, rats are preferred due to the fact that they have long been utilized for development of antiepileptics. It is further to be noted herein that the following description will be made mainly by taking rats as an example for brevity of explanation, but the present invention is not limted to rats.

At present, animal models for dieseases with genetic abnormality can be produced generally by genetic recombinant animal production techniques commonly used in the art. For the present invention, the human epilepsy model animals involved in human autosomal dominant nocturnal frontal lobe epilepsy that is one form of familial epilepsy and is characterized by nocturnal epileptic seizures can be produced by recombinant animal production techniques that have heretofore been conventionally used in the involved technical field.

At this end, a homologous gene of the α4 subunit (*CHRNA4*) or β2 subunit *(CHRNB2)* of the neuronal nicotinic acetylcholinergic receptor gene associated with human autosomal dominant nocturnal frontal lobe epilepsy is isolated from rats, etc. by techniques as used conventionally in the art, such as PCR cloning, or the like. The amino acid sequence of rat *Chrna4* cDNA is deposited under NM_000744 (NCBI) of human *CHRNA4* cDNA database L31620 and the base sequence of rat *Chrnb2* is deposited under NM_019297 (NCBI).

The base sequence of the α4 subunit (*Chrna4*) of the rat neuronal nicotinic acetylcholinergic receptor gene (wild type) is illustrated as indicated immediately below. The underlined portion indicates the portion to be substituted for a probe in accordance with the present invention. The corresponding amino acid sequence is indicated as SEQ ID No. 30.

### (SEQ ID No. 1)

ATGGCCAATTCGGGCACCGGGGCGCCGCCGCTGCTGCTACTGCCGCTGCTGCTGCTCCTA
GGGACCGGCCTCTTGCCTGCTAGCAGCCACATAGAGACCCGGGCCCATGCGGAGGAGCGGCTC
CTGAAGAGACTCTTCTCCGGTTACAACAAGTGGTCTCGGCCAGTAGCCAATATCTCAGATGTG
GTCCTCGTCCGCTTTGGCTTGTCCATTGCTCAGCTCATTGACGTGGACGAGAAGAACCAGATG
ATGACAACCAACGTGTGGGTGAAGCAGGAGTGGCACGACTACAAGCTGCGCTGGGACCCTGGT
GACTACGAGAATGTCACCTCCATCCGCATCCCCTCTGAACTCATCTGGAGGCCTGACATCGTC
CTCTACAACAATGCGGATGGAGACTTTGCAGTCACCCACCTGACCAAGGCCCACCTGTTCTAT
GACGGAAGGGTGCAGTGGACACCCCCAGCCATCTATAAGAGCTCCTGCAGCATCGACGTCACC
TTCTTCCCCTTTGACCAGCAGAACTGTACCATGAAGTTTGGATCCTGGACCTACGACAAGGCC
AAGATTGACTTAGTGAGCATGCATAGCCGTGTGGACCAACTGGACTTCTGGAAAGTGGGGAG
TGGGTCATCGTGGATGCTGTGGGCACCTACAACACCAGGAAGTACGAGTGCTGTGCCGAGATC
TATCCTGACATCACCTATGCCTTCATCATCCGACGGCTGCCGCTATTCTACACCATCAACCTC
ATCATCCCGTGCCTGCTCATCTCCTGTCTCACCGTGCTGGTCTTCTATCTGCCTTCAGAGTGT
GGCGAGAAGGTCACACTGTGCATCTCGGTGCTGCTTTCTCTCACCGTCTTCCTGCTGCTCATC
ACCGAGATCATCCCGTCCACCTCGCTGGTCATCCCGCTCATCGGCGAGTACCTCCTCTTCACC
ATGATCTTCGTCACCCTCTCCATCGTCATCACGGTCTTCGTGCTCAATGTGCACCACCGCTCG
CCACGCACACACACGATGCCCGCCTGGGTGCGTAGAGTCTTCCTGGACATCGTGCCTCGCCTC
CTCTTCATGAAGCGCCCCTCTGTGGTCAAAGACAACTGCCGGAGACTTATTGAGTCCATGCAC
AAGATGGCCAACGCCCCCCGCTTCTGGCCAGAGCCTGTGGGCGAGCCCGGCATCTTGAGTGAC
ATCTGCAACCAAGGTCTGTCACCTGCCCCAACTTTCTGCAACCCCACGGACACAGCAGTCGAG
ACCCAGCCTACGTGCAGGTCACCCCCCCTTGAGGTCCCTGACTTGAAGACATCAGAGGTTGAG
AAGGCCAGTCCCTGTCCATCGCCTGGCTCCTGTCCTCCACCCAAGAGCAGCAGTGGGGCTCCA
ATGCTCATCAAAGCCAGGTCCCTGAGTGTCCAGCATGTGCCCAGCTCCCAAGAAGCAGCAGAA

GATGGCATCCGCTGCCGGTCTCGGAGTATCCAGTACTGTGTTTCCCAAGATGGAGCTGCCTCC
CTGGCTGACAGCAAGCCCACCAGCTCCCCGACCTCCCTGAAGGCCCGTCCATCCCAGCTTCCC
GTGTCAGACCAGGCCTCTCCATGCAAATGCACATGCAAGGAACCATCTCCTGTGTCCCCAGTC
ACTGTGCTCAAGGCGGGAGGCACCAAAGCACCTCCCCAACACCTGCCCCTGTCACCAGCCCTG
ACACGGGCAGTAGAAGGCGTCCAGTACATTGCAGACCACCTCAAGGCAGAAGACACTGACTTC
TCGGTGAAGGAGGACTGGAAATACGTGGCCATGGTCATTGACCGAATCTTCCTCTGGATGTTC
ATCATTGTCTGCCTTCTGGGCACTGTGGGACTCTTCCTGCCTCCCTGGCTGGCTGCTTGCTGA

The base sequence of the β2 subunit (*Chrnb2*) of the rat neuronal nicotinic acetylcholinergic receptor gene (wild type) is illustrated as indicated immediately below. The underlined portion indicates the portion to be substituted for a probe in accordance with the present invention. The corresponding amino acid sequence is indicated as SEQ ID No. 31.

### (SEQ ID No. 2)

ATGGCCGGGCACTCCAACTCAATGGCGCTGTTCAGCTTCAGCCTTCTTTGGCTGTGCTCAGGG
GTTTTGGGAACTGACACAGAGGAGCGGCTAGTGGAGCATCTCTTAGATCCTCCCGCTATAAC
AAGCTGATTCGTCCAGCTACTAACGGCTCTGAGCTGGTGACTGTACAGCTCATGGTATCATTG
GCTCAGCTCATTAGTGTGCACGAGCGGGAGCAGATCATGACCACCAATGTCTGGCTGACCCAG
GAGTGGGAAGATTACCGCCTCACATGGAAGCCTGAGGACTTCGACAATATGAAGAAAGTCCGG
CTCCCTTCCAAACACATCTGGCTCCCAGATGTGGTTCTATACAACAATGCTGACGGCATGTAC
GAAGTCTCCTTCTATTCCAATGCTGTGGTCTCCTATGATGGCAGCATCTTTTGGCTACCACCT
GCCATCTACAAGAGTGCATGCAAGATTGAGGTGAAGCACTTCCCATTTGACCAGCAGAATTGC
ACCATGAAGTTTCGCTCATGGACCTACGACCGTACTGAGATTGACCTGGTGCTCAAAAGTGAT
GTGGCCAGTCTGGATGACTTCACACCCAGCGGGGAGTGGGACATCATCGCACTGCCAGGCCGA
CGCAACGAGAACCCAGACGACTCCACCTATGTGGACATCACCTATGACTTCATCATTCGTCGC
AAACCACTCTTCTACACTATCAACCTCATCATCCCCTGCGTACTCATCACCTCGCTGGCCATC
CTGGTCTTCTACCTGCCCTCAGACTGTGGTGAAAAGATGACACTTTGTATTTCTGTGCTGCTA
GCACTCACGGTGTTCCTGCTGCTCATCTCCAAGATTGTGCCTCCCACCTCCCTCGATGTACCG
CTGGTGGGCAAGTACCTCATGTTTACCATGGTGCTAGTCACCTTCTCCATCGTCACCAGCGTG
TGTGTGCTCAATGTGCACCACCGCTCGCCTACCACGCACACCATGGCCCCCTGGGTCAAGGTG

GTCTTCCTGGAGAAGCTGCCCACCCTGCTCTTCCTGCAGCAGCCACGCCACCGCTGTGCACGT
CAGCGTCTGCGCTTGAGGAGGCGCCAGCGAGAGCGTGAGGGCGCAGGCGCGCTTTTCTTCCGT
GAAGGTCCTGCGGCTGACCCATGTACCTGCTTTGTCAACCCTGCATCAGTGCAGGGCTTGGCT
GGGGCTTTCCGAGCTGAGCCCACTGCAGCCGGCCCGGGGCGCTCTGTGGGGCCATGCAGCTGT
GGCCTCCGGGAAGCAGTGGATGGCGTACGCTTCATTGCGGACCACATGCGAAGTGAGGATGAT
GACCAGAGTGTGAGGGAGGACTGGAAATACGTTGCCATGGTGATCGACCGCCTGTTCCTGTGG
ATCTTTGTCTTTGTCTGTGTCTTTGGGACCGTCGGCATGTTCCTGCAGCCTCTCTTCCAGAAC
TACACTGCCACTACCTTCCTCCACCCTGACCACTCAGCTCCCAGCTCCAAGTGA

The cDNA clones as prepared above may be subjected to mutation in accordance with mutation techniques known to the art. As the mutation techniques to be used for the present invention, there may be used conventional ones as have been frequently used in the art, such as site-directed mutagenesis. The site-directed mutagenesis allows an optional mutation to be inserted site-specifically into an optional site of a cDNA. The mutation to be site-specifically inserted into the optional site of the cDNA is not limited to a specific one and may include modifications, for example, such as deletion, defect, substitution, and addition.

Therefore, the present invention enables a specific mutation to be inserted into an isolated rat *Chrna4* or *Chrnb2* using the site-directed mutagenesis, resulting in the rat homologous gene *Chrna4* or *Chrnb2* with the mutation inserted therein, respectively, which possesses a base sequence coding for a protein having functions associated with the onset of nocturnal epileptic seizures derived from human autosomal dominant nocturnal frontal lobe epilepsy.

As used herein, the term "homologous gene" or the terms relating thereto refers to a gene which has a base sequence equivalent of that of a gene derived from an animal of a different species considered as having a common ancestor and which encodes a protein having equivalent functions.
In accordance with the present invention, the cDNA of the subunits of the rat neuronal nicotinic acetylcholinergic receptor gene may be prepared in the manner as will be described hereinafter.

More specifically, a set consisting of two kinds of primers, for example, a forward primer and a reverse primer, each having the following base sequence, is designed and prepared on the basis of the cDNA sequence of the known rat *Chrna4* or *Chrnb2* using the rat cDNA clone, respectively, as a template.

The base sequences of the forward primer (40mer) (SEQ ID No. 3) and the reverse primer (38mer) (SEQ ID No. 4), each prepared, respectively, on the basis of the cDNA sequence of the rat *Chrna4,* are indicated as below:

SEQ ID No. 3:AGATCTCGCGAAGCTTCACCATGGCCAATTCGGGCACCGG
SEQ ID No. 4:AGATCTAGATCAGCAAGCAGCCAGCCAGGGAGGCAGGA
The base sequences of the forward primer (41mer) (SEQ ID No. 5) and the reverse primer (40mer) (SEQ ID No. 6), each prepared, respectively, on the basis of the cDNA sequence of the rat *Chrnb2,* are indicated as below:

SEQ ID No. 5:AGATCTCGCGACATGGCCGGGCACTCCAACTCAATGGCGCT
SEQ ID No. 6:ATCGATGGATCCTCACTTGGAGCTGGGAGCTGAGTGGTCA
The PCR is performed using these primers and the resulting PCR products are sub-cloned in an appropriate vector. The resulting clones are then sequenced to confirm the base sequences of the *Chrna4* and *Chrnb2* cDNAs.

Thereafter, a mutation is inserted into an optional site of the resulting cDNA clones as obtained above. The mutation may be performed using various mutation techniques known to the art. An optional mutation may be introduced into an optional site using known mutation techniques in particular including site-directed mutagenesis.

As previously described, three mutations, i.e., S280F, S284L, and 291-292insL are reported as gene abnormality of the *CHRNA4* gene. Three mutations, i.e., V287L, V287M, and I312M are reported as genetic mutations of the *CHRNB2* gene.

Therefore, the present invention allows a mutation of a species-specific homologous gene corresponding to, for example, the mutations S280F, S284L or 291-292insL of the rat *CHRNA4* gene or the mutations V287L, V287M or I312M of the rat *CHRNB2* gene in the rat *Chrna4* (wild type) (SEQ ID NO.1) and the rat *Chrnb2* (wild type) (SEQ ID NO.2), respectively, isolated by the PCR cloning as performed above, to be performed by the above mutation techniques.

Thus, the mutation may be performed to introduce a specific mutation into a specific mutation site with a commercially available kit, using appropriate sense primers and anti-sense primers. The sense primer and anti-sense primer to be used for the mutation may be in the form of generally 20mer to 40mer, preferably 25mer to 35mer. In this case, it is preferred to design the mutation site and perform the mutation so as to allow a restriction site of a restriction enzyme to appear on a mutation site.

More specifically, for instance, the mutation S282F (i.e., S280F in humans) may be introduced in the wild-type rat *Chrna4* using a sense primer (29mer) and an anti-sense primer (29mer) as indicated below to replace cytosine (C) at cDNA base sequence position 845 by thymine (T) (c.845C>T) and guanidine (G) at cDNA base sequence position 846 by cytosine (C) (c.846G>C). This mutation allows the amino acid residue Ser homologous to position 282 of *CHRNA4* to be replaced by Phe.

The base sequences of the sense primer (SEQ ID No. 7) and the anti-sense primer (SEQ ID No. 8) to be used herein are indicated as follows:

SEQ ID No. 7:CACACTGTGCATCTTCGTGCTGCTTTCTC
SEQ ID No. 8:GAGAAAGCAGCACGAAGATGCACAGTGTG
The base sequence of the resulting mutated rat cDNA is indicated as follows:

### (SEQ ID No. 9)

ATGGCCAATTCGGGCACCGGGGCGCCGCCGCTGCTGCTACTGCCGCTGCTGCTGCTCCTA
GGGACCGGCCTCTTGCCTGCTAGCAGCCACATAGAGACCCGGCCCATGCGGAGGAGCGGCTCC
TGAAGAGACTCTTCTCCGGTTACAACAAGTGGTCTCGGCCAGTAGCCAATATCTCAGATGTGG
TCCTCGTCCGCTTTGGCTTGTCCATTGCTCAGCTCATTGACGTGGACGAGAAGAACCAGATGA

TGACAACCAACGTGTGGGTGAAGCAGGAGTGGCACGACTACAAGCTGCGCTGGGACCCTGGTG
ACTACGAGAATGTCACCTCCATCCGCATCCCCTCTGAACTCATCTGGAGGCCTGACATCGTCC
TCTACAACAATGCGGATGGAGACTTTGCAGTCACCCACCTGACCAAGGCCCACCTGTTCTATG
ACGGAAGGGTGCAGTGGACACCCCCAGCCATCTATAAGAGCTCCTGCAGCATCGACGTCACCT
TCTTCCCCTTTGACCAGCAGAACTGTACCATGAAGTTTGGATCCTGGACCTACGACAAGGCCA
AGATTGACTTAGTGAGCATGCATAGCCGTGTGGACCAACTGGACTTCTGGGAAAGTGGGGAGT
GGGTCATCGTGGATGCTGTGGGCACCTACAACACCAGGAAGTACGAGTGCTGTGCCGAGATCT
ATCCTGACATCACCTATGCCTTCATCATCCGACGGCTGCCGCTATTCTACACCATCAACCTCA
TCATCCCGTGCCTGCTCATCTCCTGTCTCACCGTGCTGGTCTTCTATCTGCCTTCAGAGTGTG
GCGAGAAGGTCACACTGTGCATCTTCGTGCTGCTTTCTCTCACCGTCTTCCTGCTGCTCATCA
CCGAGATCATCCCGTCCACCTCGCTGGTCATCCCGCTCATCGGCGAGTACCTCCTCTTCACCA
TGATCTTCGTCACCCTCTCCATCGTCATCACGGTCTTCGTGCTCAATGTGCACCACCGCTCGC
CACGCACACACACGATGCCCGCCTGGGTGCGTAGAGTCTTCCTGGACATCGTGCCTCGCCTCC
TCTTCATGAAGCGCCCCTCTGTGGTCAAAGACAACTGCCGGAGACTTATTGAGTCCATGCACA
AGATGGCCAACGCCCCCCGCTTCTGGCCAGAGCCTGTGGGCGAGCCCGGCATCTTGAGTGACA
TCTGCAACCAAGGTCTGTCACCTGCCCCAACTTTCTGCAACCCCACGGACACAGCAGTCGAGA
CCCAGCCTACGTGCAGGTCACCCCCCCTTGAGGTCCCTGACTTGAAGACATCAGAGGTTGAGA
AGGCCAGTCCCTGTCCATCGCCTGGCTCCTGTCCTCCACCCAAGAGCAGCAGTGGGGCTCCAA
TGCTCATCAAAGCCAGGTCCCTGAGTGTCCAGCATGTGCCCAGCTCCCAAGAAGCAGCAGAAG
ATGGCATCCGCTGCCGGTCTCGGAGTATCCAGTACTGTGTTTCCCAAGATGGAGCTGCCTCCC
TGGCTGACAGCAAGCCCACCAGCTCCCCGACCTCCCTGAAGGCCCGTCCATCCCAGCTTCCCG
TGTCAGACCAGGCCTCTCCATGCAAATGCACATGCAAGGAACCATCTCCTGTGTCCCCAGTCA
CTGTGCTCAAGGCGGGAGGCACCAAAGCACCTCCCCAACACCTGCCCCTGTCACCAGCCCTGA
CACGGGCAGTAGAAGGCGTCCAGTACATTGCAGACCACCTCAAGGCAGAAGACACTGACTTCT
CGGTGAAGGAGGACTGGAAATACGTGGCCATGGTCATTGACCGAATCTTCCTCTGGATGTTCA
TCATTGTCTGCCTTCTGGGCACTGTGGGACTCTTCCTGCCTCCCTGGCTGGCTGCTTGCTGA

Similarly, the mutation of S286L (i.e., S284L in humans) may be introduced into the wild-type rat *Chrna4* using a sense primer (29mer) and an anti-sense primer (29mer) as indicated below to replace tyrosine (T) at base sequence position 856 of cDNA by cysteine (C) (c.856T>C) and cysteine (C) at base sequence position 857 thereof by tyrosine (T) (c.857C>T). This mutation results in replacement of the amino acid residue Ser homologous to position 286 of *CHRNA4* by the amino acid residue Leu.

The base sequences of the sense primer (SEQ ID No. 10) and the anti-sense primer (SEQ ID No. 11) to be used herein are indicated as follows:

SEQ ID No. 10:CGGTGCTGCTTCTTCTCACCGTCTTCCTG
SEQ ID No. 11:CAGGAAGACGGTGAGAAGAAGCAGCACCG
The base sequence of the resulting mutated rat cDNA is indicated as follows:

### (SEQ ID No. 12)

ATGGCCAATTCGGGCACCGGGGCGCCGCCGCTGCTGCTACTGCCGCTGCTGCTGCTCCTA
GGGACCGGCCTCTTGCCTGCTAGCAGCCACATAGAGACCCGGGCCCATGCGGAGGAGCGGCTC
CTGAAGAGACTCTTCTCCGGTTACAACAAGTGGTCTCGGCCAGTAGCCAATATCTCAGATGTG
GTCCTCGTCCGCTTTGGCTTGTCCATTGCTCAGCTCATTGACGTGGACGAGAAGAACCAGATG
ATGACAACCAACGTGTGGGTGAAGCAGGAGTGGCACGACTACAAGCTGCGCTGGGACCCTGGT
GACTACGAGAATGTCACCTCCATCCGCATCCCCTCTGAACTCATCTGGAGGCCTGACATCGTC
CTCTACAACAATGCGGATGGAGACTTTGCAGTCACCCACCTGACCAAGGCCCACCTGTTCTAT
GACGGAAGGGTGCAGTGGACACCCCCAGCCATCTATAAGAGCTCCTGCAGCATCGACGTCACC
TTCTTCCCCTTTGACCAGCAGAACTGTACCATGAAGTTTGGATCCTGGACCTACGACAAGGCC
AAGATTGACTTAGTGAGCATGCATAGCCGTGTGGACCAACTGGACTTCTGGAAAGTGGGGAG
TGGGTCATCGTGGATGCTGTGGGCACCTACAACACCAGGAAGTACGAGTGCTGTGCCGAGATC
TATCCTGACATCACCTATGCCTTCATCATCCGACGGCTGCCGCTATTCTACACCATCAACCTC
ATCATCCCGTGCCTGCTCATCTCCTGTCTCACCGTGCTGGTCTTCTATCTGCCTTCAGAGTGT
GGCGAGAAGGTCACACTGTGCATCTCGGTGCTGCTTCTTCTCACCGTCTTCCTGCTGCTCATC
ACCGAGATCATCCCGTCCACCTCGCTGGTCATCCCGCTCATCGGCGAGTACCTCCTCTTCACC

ATGATCTTCGTCACCCTCTCCATCGTCATCACGGTCTTCGTGCTCAATGTGCACCACCGCTCG
CCACGCACACACACGATGCCCGCCTGGGTGCGTAGAGTCTTCCTGGACATCGTGCCTCGCCTC
CTCTTCATGAAGCGCCCCTCTGTGGTCAAAGACAACTGCCGGAGACTTATTGAGTCCATGCAC
AAGATGGCCAACGCCCCCCGCTTCTGGCCAGAGCCTGTGGGCGAGCCCGGCATCTTGAGTGAC
ATCTGCAACCAAGGTCTGTCACCTGCCCCAACTTTCTGCAACCCCACGGACACAGCAGTCGAG
ACCCAGCCTACGTGCAGGTCACCCCCCCTTGAGGTCCCTGACTTGAAGACATCAGAGGTTGAG
AAGGCCAGTCCCTGTCCATCGCCTGGCTCCTGTCCTCCACCCAAGAGCAGCAGTGGGGCTCCA
ATGCTCATCAAAGCCAGGTCCCTGAGTGTCCAGCATGTGCCCAGCTCCCAAGAAGCAGCAGAA
GATGGCATCCGCTGCCGGTCTCGGAGTATCCAGTACTGTGTTTCCCAAGATGGAGCTGCCTCC
CTGGCTGACAGCAAGCCCACCAGCTCCCCGACCTCCCTGAAGGCCCGTCCATCCCAGCTTCCC
GTGTCAGACCAGGCCTCTCCATGCAAATGCACATGCAAGGAACCATCTCCTGTGTCCCCAGTC
ACTGTGCTCAAGGCGGAGGCACCAAAGCACCTCCCCAACACCTGCCCCTGTCACCAGCCCTG
ACACGGGCAGTAGAAGGCGTCCAGTACATTGCAGACCACCTCAAGGCAGAAGACACTGACTTC
TCGGTGAAGGAGGACTGGAAATACGTGGCCATGGTCATTGACCGAATCTTCCTCTGGATGTTC
ATCATTGTCTGCCTTCTGGGCACTGTGGGACTCTTCCTGCCTCCCTGGCTGGCTGCTTGCTGA

In a manner similar to the above mutation, the mutated bases GCT (c.878-879insGCT) may be inserted in between the bases at positions 878 and 879 of the wild-type rat *Chrna4* cDNA, thereby allowing an insertion of the amino acid residue leucine (Leu) to be inserted in between the amino acid residue leucine (Leu) homologous to the amino acid at position 293 (i.e., 291 in humans) of *CHRNA4* and the amino acid residue isoleucine (Ile) homologous to the amino acid at position 294 (i.e., 292 in humans) thereof using a sense primer (30mer) and an antisense primer (30mer) as indicated below.

The base sequences of the sense primer (SEQ ID No. 13) and the anti-sense primer (SEQ ID No. 14) are indicated as follows:

SEQ ID No. 13:GTCTTCCTGCTGCTGCTCATCACCGAGATC
SEQ ID No. 14:GATCTCGGTGATGAGCAGCAGCAGGAAGAC
The base sequence of the cDNA resulting by the above mutation is indicated as follows:

(SEQ ID No. 15)
ATGGCCAATTCGGGCACCGGGGCGCCGCCGCTGCTGCTACTGCCGCTGCTGCTGCTCCTA
GGGACCGGCCTCTTGCCTGCTAGCAGCCACATAGAGACCCGGGCCCATGCGGAGGAGCGGCTC
CTGAAGAGACTCTTCTCCGGTTACAACAAGTGGTCTCGGCCAGTAGCCAATATCTCAGATGTG
GTCCTCGTCCGCTTTGGCTTGTCCATTGCTCAGCTCATTGACGTGGACGAGAAGAACCAGATG
ATGACAACCAACGTGTGGGTGAAGCAGGAGTGGCACGACTACAAGCTGCGCTGGGACCCTGGT
GACTACGAGAATGTCACCTCCATCCGCATCCCCTCTGAACTCATCTGGAGGCCTGACATCGTC
CTCTACAACAATGCGGATGGAGACTTTGCAGTCACCCACCTGACCAAGGCCCACCTGTTCTAT
GACGGAAGGGTGCAGTGGACACCCCCAGCCATCTATAAGAGCTCCTGCAGCATCGACGTCACC
TTCTTCCCCTTTGACCAGCAGAACTGTACCATGAAGTTTGGATCCTGGACCTACGACAAGGCC
AAGATTGACTTAGTGAGCATGCATAGCCGTGTGGACCAACTGGACTTCTGGGAAAGTGGGGAG
TGGGTCATCGTGGATGCTGTGGGCACCTACAACACCAGGAAGTACGAGTGCTGTGCCGAGATC
TATCCTGACATCACCTATGCCTTCATCATCCGACGGCTGCCGCTATTCTACACCATCAACCTC
ATCATCCCGTGCCTGCTCATCTCCTGTCTCACCGTGCTGGTCTTCTATCTGCCTTCAGAGTGT
GGCGAGAAGGTCACACTGTGCATCTCGGTGCTGCTTTCTCTCACCGTCTTCCTGCTGCTA
ATCACCGAGATCATCCCGTCCACCTCGCTGGTCATCCCGCTCATCGGCGAGTACCTCCTCTTC
ACCATGATCTTCGTCACCCTCTCCATCGTCATCACGGTCTTCGTGCTCAATGTGCACCACCGC
TCGCCACGCACACACACGATGCCCGCCTGGGTGCGTAGAGTCTTCCTGGACATCGTGCCTCGC
CTCCTCTTCATGAAGCGCCCCTCTGTGGTCAAAGACAACTGCCGGAGACTTATTGAGTCCATG
CACAAGATGGCCAACGCCCCCCGCTTCTGGCCAGAGCCTGTGGGCGAGCCCGGCATCTTGAGT
GACATCTGCAACCAAGGTCTGTCACCTGCCCCAACTTTCTGCAACCCCACGGACACAGCAGTC
GAGACCCAGCCTACGTGCAGGTCACCCCCCCTTGAGGTCCCTGACTTGAAGACATCAGAGGTT
GAGAAGGCCAGTCCCTGTCCATCGCCTGGCTCCTGTCCTCCACCCAAGAGCAGCAGTGGGGCT
CCAATGCTCATCAAAGCCAGGTCCCTGAGTGTCCAGCATGTGCCCAGCTCCCAAGAAGCAGCA
GAAGATGGCATCCGCTGCCGGTCTCGGAGTATCCAGTACTGTGTTTCCCAAGATGGAGCTGCC
TCCCTGGCTGACAGCAAGCCCACCAGCTCCCCGACCTCCCTGAAGGCCCGTCCATCCCAGCTT

CCCGTGTCAGACCAGGCCTCTCCATGCAAATGCACATGCAAGGAACCATCTCCTGTGTCCCCA
GTCACTGTGCTCAAGGCGGAGGCACCAAAGCACCTCCCCAACACCTGCCCCTGTCACCAGCC
CTGACACGGGCAGTAGAAGGCGTCCAGTACATTGCAGACCACCTCAAGGCAGAAGACACTGAC
TTCTCGGTGAAGGAGGACTGGAAATACGTGGCCATGGTCATTGACCGAATCTTCCTCTGGATG
TTCATCATTGTCTGCCTTCTGGGCACTGTGGGACTCTTCCTGCCTCCCTGGCTGGCTGCTTGC
TGA

In substantially the same manner as in the case of the above c.878-879insGCT, the bases TTA (c.879-880insTTA) may be inserted in between the bases at positions 879 and 880 of the wild-type rat *Chrna4* cDNA, resulting in an insertion of the amino acid residue leucine (Leu) in between the amino acid residue leucine (Leu) homologous to the amino acid at position 293 (i.e., 291 in humans) of *CHRNA4* and the amino acid residue isoleucine (Ile) homologous to the amino acid at position 294 (i.e.,292 in humans) thereof.

### (SEQ ID No. 16)

ATGGCCAATTCGGGCACCGGGGCGCCGCCGCTGCTGCTACTGCCGCTGCTGCTGCTCCTA
GGGACCGGCCTCTTGCCTGCTAGCAGCCACATAGAGACCCGGGCCCATGCGGAGGAGCGGCTC
CTGAAGAGACTCTTCTCCGGTTACAACAAGTGGTCTCGGCCAGTAGCCAATATCTCAGATGTG
GTCCTCGTCCGCTTTGGCTTGTCCATTGCTCAGCTCATTGACGTGGACGAGAAGAACCAGATG
ATGACAACCAACGTGTGGGTGAAGCAGGAGTGGCACGACTACAAGCTGCGCTGGGACCCTGGT
GACTACGAGAATGTCACCTCCATCCGCATCCCCTCTGAACTCATCTGGAGGCCTGACATCGTC
CTCTACAACAATGCGGATGGAGACTTTGCAGTCACCCACCTGACCAAGGCCCACCTGTTCTAT
GACGGAAGGGTGCAGTGGACACCCCCAGCCATCTATAAGAGCTCCTGCAGCATCGACGTCACC
TTCTTCCCCTTTGACCAGCAGAACTGTACCATGAAGTTTGGATCCTGGACCTACGACAAGGCC
AAGATTGACTTAGTGAGCATGCATAGCCGTGTGGACCAACTGGACTTCTGGGAAAGTGGGGAG
TGGGTCATCGTGGATGCTGTGGGCACCTACAACACCAGGAAGTACGAGTGCTGTGCCGAGATC
TATCCTGACATCACCTATGCCTTCATCATCCGACGGCTGCCGCTATTCTACACCATCAACCTC
ATCATCCCGTGCCTGCTCATCTCCTGTCTCACCGTGCTGGTCTTCTATCTGCCTTCAGAGTGT
GGCGAGAAGGTCACACTGTGCATCTCGGTGCTGCTTTCTCTCACCGTCTTCCTGCTGCTCTTA

ATCACCGAGATCATCCCGTCCACCTCGCTGGTCATCCCGCTCATCGGCGAGTACCTCCTCTTC
ACCATGATCTTCGTCACCCTCTCCATCGTCATCACGGTCTTCGTGCTCAATGTGCACCACCGC
TCGCCACGCACACACACGATGCCCGCCTGGGTGCGTAGAGTCTTCCTGGACATCGTGCCTCGC
CTCCTCTTCATGAAGCGCCCCTCTGTGGTCAAAGACAACTGCCGGAGACTTATTGAGTCCATG
CACAAGATGGCCAACGCCCCCCGCTTCTGGCCAGAGCCTGTGGGCGAGCCCGGCATCTTGAGT
GACATCTGCAACCAAGGTCTGTCACCTGCCCCAACTTTCTGCAACCCCACGGACACAGCAGTC
GAGACCCAGCCTACGTGCAGGTCACCCCCCCTTGAGGTCCCTGACTTGAAGACATCAGAGGTT
GAGAAGGCCAGTCCCTGTCCATCGCCTGGCTCCTGTCCTCCACCCAAGAGCAGCAGTGGGGCT
CCAATGCTCATCAAAGCCAGGTCCCTGAGTGTCCAGCATGTGCCCAGCTCCCAAGAAGCAGCA
GAAGATGGCATCCGCTGCCGGTCTCGGAGTATCCAGTACTGTGTTTCCCAAGATGGAGCTGCC
TCCCTGGCTGACAGCAAGCCCACCAGCTCCCCGACCTCCCTGAAGGCCCGTCCATCCCAGCTT
CCCGTGTCAGACCAGGCCTCTCCATGCAAATGCACATGCAAGGAACCATCTCCTGTGTCCCCA
GTCACTGTGCTCAAGGCGGGAGGCACCAAAGCACCTCCCCAACACCTGCCCCTGTCACCAGCC
CTGACACGGGCAGTAGAAGGCGTCCAGTACATTGCAGACCACCTCAAGGCAGAAGACACTGAC
TTCTCGGTGAAGGAGGACTGGAAATACGTGGCCATGGTCATTGACCGAATCTTCCTCTGGATG
TTCATCATTGTCTGCCTTCTGGGCACTGTGGGACTCTTCCTGCCTCCCTGGCTGGCTGCTTGC
TGA

Similarly, by inserting the mutation V287L in the wild-type rat *Chrnb2,* the base (G) at position c.856 is replaced by the base (C) (c.856G>C) using a forward primer (SEQ ID No. 17) and a reverse primer (SEQ ID No. 18) as indicated below. This results in substitution of the amino acid residue (Val) at position 286 homologous to the β2 subunit of human neuronal nicotinic acetylcholinergic receptor gene for the amino acid residue Leu.

The base sequences of the forward primer (30mer) (SEQ ID No. 17) and the reverse primer (30mer) (SEQ ID No. 18) are indicated as follows:

SEQ ID No. 17:CTCATCTCCAAGATTATGCCTCCCACCTCC
SEQ ID No. 18:GGAGGTGGGAGGCATAATCTTGGAGATGAG
   The base sequence of the cDNA obtained by the above mutation is indicated as follows:

### (SEQ ID No. 19)

GGCCGGGCACTCCAACTCAATGGCGCTGTTCAGCTTCAGCCTTCTTTGGCTGTGCTCAGGGGT
TTTGGGAACTGACACAGAGGAGCGGCTAGTGGAGCATCTCTTAGATCCCTCCCGCTATAACAA
GCTGATTCGTCCAGCTACTAACGGCTCTGAGCTGGTGACTGTACAGCTCATGGTATCATTGGC
TCAGCTCATTAGTGTGCACGAGCGGGAGCAGATCATGACCACCAATGTCTGGCTGACCCAGGA
GTGGGAAGATTACCGCCTCACATGGAAGCCTGAGGACTTCGACAATATGAAGAAAGTCCGGCT
CCCTTCCAAACACATCTGGCTCCCAGATGTGGTTCTATACAACAATGCTGACGGCATGTACGA
AGTCTCCTTCTATTCCAATGCTGTGGTCTCCTATGATGGCAGCATCTTTTGGCTACCACCTGC
CATCTACAAGAGTGCATGCAAGATTGAGGTGAAGCACTTCCCATTTGACCAGCAGAATTGCAC
CATGAAGTTTCGCTCATGGACCTACGACCGTACTGAGATTGACCTGGTGCTCAAAAGTGATGT
GGCCAGTCTGGATGACTTCACACCCAGCGGGGAGTGGACATCATCGCACTGCCAGGCCGACG
CAACGAGAACCCAGACGACTCCACCTATGTGGACATCACCTATGACTTCATCATTCGTCGCAA
ACCACTCTTCTACACTATCAACCTCATCATCCCCTGCGTACTCATCACCTCGCTGGCCATCCT
GGTCTTCTACCTGCCCTCAGACTGTGGTGAAAAGATGACACTTTGTATTTCTGTGCTGCTAGC
ACTCACGGTGTTCCTGCTGCTCATCTCCAAGATTCTGCCTCCCACCTCCCTCGATGTACCGCT
GGTGGGCAAGTACCTCATGTTTACCATGGTGCTAGTCACCTTCTCCATCGTCACCAGCGTGTG
TGTGCTCAATGTGCACCACCGCTCGCCTACCACGCACACCATGGCCCCCTGGGTCAAGGTGGT
CTTCCTGGAGAAGCTGCCCACCCTGCTCTTCCTGCAGCAGCCACGCCACCGCTGTGCACGTCA
GCGTCTGCGCTTGAGGAGGCGCCAGCGAGAGCGTGAGGGCGCAGGCGCGCTTTTCTTCCGTGA
AGGTCCTGCGGCTGACCCATGTACCTGCTTTGTCAACCCTGCATCAGTGCAGGGCTTGGCTGG
GGCTTTCCGAGCTGAGCCCACTGCAGCCGGCCCGGGGCGCTCTGTGGGGCCATGCAGCTGTGG
CCTCCGGGAAGCAGTGGATGGCGTACGCTTCATTGCGGACCACATGCGAAGTGAGGATGATGA
CCAGAGTGTGAGGGAGGACTGGAAATACGTTGCCATGGTGATCGACCGCCTGTTCCTGTGGAT
CTTTGTCTTTGTCTGTGTCTTTGGGACCGTCGGCATGTTCCTGCAGCCTCTCTTCCAGAACTA
CACTGCCACTACCTTCCTCCACCCTGACCACTCAGCTCCCAGCTCCAAGTGA

In a manner similar to the above mutation, the base residue guanine (G) at position c.856 may be replaced by the base residue adenine (A) (c.856G>A) by inserting the mutation V287M in the wild-type rat *Chrnb2*, using a forward primer (SEQ ID No. 20) and a reverse primer (SEQ ID No. 21) as indicated below. This insertion results in substitution of the amino acid residue (Val) at position 286 homologous to the β2 subunit of human neuronal nicotinic acetylcholinergic receptor gene for the amino acid residue Met.

The base sequences of the forward primer (30mer) (SEQ ID No. 20) and the reverse primer (30mer) (SEQ ID No. 21) usable for the present invention are indicated as follows:

SEQ ID No. 20:CTCATCTCCAAGATTCTGCCTCCCACCTCC
SEQ ID No. 22:GGAGGTGGGAGGCAGAATCTTGGAGATGAG
   The base sequence of the cDNA obtained by the above mutation is indicated as follows:

### (SEQ ID No. 22)

TGGCCGGGCACTCCAACTCAATGGCGCTGTTCAGCTTCAGCCTTCTTTGGCTGTGCTCAGGGG
TTTTGGGAACTGACACAGAGGAGCGGCTAGTGGAGCATCTCTTAGATCCCTCCCGCTATAACA
AGCTGATTCGTCCAGCTACTAACGGCTCTGAGCTGGTGACTGTACAGCTCATGGTATCATTGG
CTCAGCTCATTAGTGTGCACGAGCGGGAGCAGATCATGACCACCAATGTCTGGCTGACCCAGG
AGTGGGAAGATTACCGCCTCACATGGAAGCCTGAGGACTTCGACAATATGAAGAAAGTCCGGC
TCCCTTCCAAACACATCTGGCTCCCAGATGTGGTTCTATACAACAATGCTGACGGCATGTACG
AAGTCTCCTTCTATTCCAATGCTGTGGTCTCCTATGATGGCAGCATCTTTTGGCTACCACCTG
CCATCTACAAGAGTGCATGCAAGATTGAGGTGAAGCACTTCCCATTTGACCAGCAGAATTGCA
CCATGAAGTTTCGCTCATGGACCTACGACCGTACTGAGATTGACCTGGTGCTCAAAAGTGATG
TGGCCAGTCTGGATGACTTCACACCCAGCGGGGAGTGGGACATCATCGCACTGCCAGGCCGAC
GCAACGAGAACCCAGACGACTCCACCTATGTGGACATCACCTATGACTTCATCATTCGTCGCA
AACCACTCTTCTACACTATCAACCTCATCATCCCCTGCGTACTCATCACCTCGCTGGCCATCC

TGGTCTTCTACCTGCCCTCAGACTGTGGTGAAAAGATGACACTTTGTATTTCTGTGCTGCTAG
CACTCACGGTGTTCCTGCTGCTCATCTCCAAGATTATGCCTCCCACCTCCCTCGATGTACCGC
TGGTGGGCAAGTACCTCATGTTTACCATGGTGCTAGTCACCTTCTCCATCGTCACCAGCGTGT
GTGTGCTCAATGTGCACCACCGCTCGCCTACCACGCACACCATGGCCCCCTGGGTCAAGGTGG
TCTTCCTGGAGAAGCTGCCCACCCTGCTCTTCCTGCAGCAGCCACGCCACCGCTGTGCACGTC
AGCGTCTGCGCTTGAGGAGGCGCCAGCGAGAGCGTGAGGGCGCAGGCGCGCTTTTCTTCCGTG
AAGGTCCTGCGGCTGACCCATGTACCTGCTTTGTCAACCCTGCATCAGTGCAGGGCTTGGCTG
GGGCTTTCCGAGCTGAGCCCACTGCAGCCGGCCCGGGCGCTCTGTGGGGCCATGCAGCTGTG
GCCTCCGGGAAGCAGTGGATGGCGTACGCTTCATTGCGGACCACATGCGAAGTGAGGATGATG
ACCAGAGTGTGAGGGAGGACTGGAAATACGTTGCCATGGTGATCGACCGCCTGTTCCTGTGGA
TCTTTGTCTTTGTCTGTGTCTTTGGGACCGTCGGCATGTTCCTGCAGCCTCTCTTCCAGAACT
ACACTGCCACTACCTTCCTCCACCCTGACCACTCAGCTCCCAGCTCCAAGTGA

The present invention allows a new enzyme site to appear on a mutation site by designing a type of a mutation of interest to be inserted so as to correspond to a base sequence of a codon located at the position of the mutation site in the manner as described above.

For instance, in the case of *Chrna4*, the mutation of the mutated 879-880insTTA causes an appearance of the cleavage site (T↓TAA) by restriction enzyme *Tru9 I* (*MseI*). In the case of *Chrnb2*, the mutation of the mutated V287L or V287M causes an appearance of the cleavage site (G↓ANTG) by restriction enzyme *Hinf If.* In the above description, the arrow symbol (↓) denotes a position of cleavage by a restriction enzyme. It is to be rioted herein that the kinds of the restriction enzyme and restriction sites are not restricted to the above ones and may be selected appropriately by changing mutations.

In accordance with the present invention, a nucleotide consisting of a particular base sequence can be introduced as a probe into the above cDNA. The probe may be preferably prepared so as to have a base sequence thoroughly or substantially thoroughly different from the original base sequence yet encode the amino acid sequence identical thereto. The base number of the probe may be in the length ranging generally from approximately 30 bp to 200 bp, preferably from approximately 50 bp to 150 bp, more preferably from approximately 60 bp to 120 bp. These nucleotides may be prepared by conventional methods including DNA synthesis, as used commonly in the art.

More specifically, for instance, for the mutated cDNA of the *Chrna4* gene, a nucleotide having the following base sequence (118 bp) can be prepared so as to have a base sequence different from the base sequence starting with position c.104 and ending at position c.221 of the cDNA, yet coding for the same amino acid sequence.

### (SEQ ID No. 23)

GGGCTCACGCCGAAGAACGCCTGCTCAAAAGGCTGTTTTCTGGCTATAATAAATGGTCCCGCC

CCGTGGCTAACATTTCCGACGTCGTGCTGGTGCGGTTCGGATTATCTATCGCTCA

Similarly, for the mutated cDNA of the *Chrnb2* gene, a nucleotide having the following base sequence (62 bp) can be prepared as a probe so as to have a base sequence different from the base sequence starting with position c.1171 and ending at position c.1232 of the cDNA, yet coding for the same amino acid sequence.

### (SEQ ID No. 24)

AACCCCGCCTCCGTCCAAGGACTCGCCGGCGCCTTTAGGGCCGAACCTACCGCCGCTGGCCC
Thereafter, the resulting nucleotide probe may be hybridized and inserted into a site of a restriction enzyme in a conventional manner. For instance, the probe indicated below as SEQ ID No. 22 may be inserted into a designated site on the mutated cDNA of the *Chrna4* subunit gene having the above base sequence, e.g., a site of *Sma I* and *Bpul102* (alternatively called as *Esp I* or *Cel II* site) of an appropriate vector such as pCRII-TOPO vector, etc., using a probe insertion method as conventionally used in the art. Alternatively, for instance, the probe indicated below as SEQ ID No. 22 may also be inserted into a designated site of the mutated cDNA of the *Chrnb2* subunit gene having the above base sequence, e.g., a site of *Hinc I* and *Sma I* of an appropriate vector such as pCRII-TOPO vector, etc., using a probe insertion method as conventionally used in the art. At each step, the base sequence is confirmed by sequencing.

The base sequence (SEQ ID No. 25) of the mutated gene cDNA (S282FPB) with the probe (SEQ ID No. 23) inserted into the mutated cDNA (S282F) (i.e., S280F in humans) of the rat *Chrna4* subunit gene is indicated as below. It is to be noted herein that the mutation of c.845C>T and c.846G>C causes the bases TCG at positions 844-846 to be mutated into the mutated bases TTC as enclosed by circle, and the probe portion (118 bp) is indicated as underlined at positions 104-221.

### (SEQ ID No. 25)

ATGGCCAATTCGGGCACCGGGGCGCCGCCGCTGCTGCTACTGCCGCTGCTGCTGCTCCTA
GGGACCGGCCTCTTGCCTGCTAGCAGCCACATAGAGACCCGGGCTCACGCCGAAGAACGCCTG
CTCAAAAGGCTGTTTTCTGGCTATAATAAATGGTCCCGCCCCGTGGCTAACATTTCCGACGTC
GTGCTGGTGCGGTTCGGATTATCTATCGCTCAGCTCATTGACGTGGACGAGAAGAACCAGATG

ATGACAACCAACGTGTGGGTGAAGCAGGAGTGGCACGACTACAAGCTGCGCTGGACCCTGGT
GACTACGAGAATGTCACCTCCATCCGCATCCCCTCTGAACTCATCTGGAGGCCTGACATCGTC
CTCTACAACAATGCGGATGGAGACTTTGCAGTCACCCACCTGACCAAGGCCCACCTGTTCTAT
GACGGAAGGGTGCAGTGGACACCCCCAGCCATCTATAAGAGCTCCTGCAGCATCGACGTCACC
TTCTTCCCCTTTGACCAGCAGAACTGTACCATGAAGTTTGGATCCTGGACCTACGACAAGGCC
AAGATTGACTTAGTGAGCATGCATAGCCGTGTGGACCAACTGGACTTCTGGGAAAGTGGGGAG
TGGGTCATCGTGGATGCTGTGGGCACCTACAACACCAGGAAGTACGAGTGCTGTGCCGAGATC
TATCCTGACATCACCTATGCCTTCATCATCCGACGGCTGCCGCTATTCTACACCATCAACCTC
ATCATCCCGTGCCTGCTCATCTCCTGTCTCACCGTGCTGGTCTTCTATCTGCCTTCAGAGTGT
GGCGAGAAGGTCACACTGTGCATCTTCGTGCTGCTTTCTCTCACCGTCTTCCTGCTGCTCATC
ACCGAGATCATCCCGTCCACCTCGCTGGTCATCCCGCTCATCGGCGAGTACCTCCTCTTCACC
ATGATCTTCGTCACCCTCTCCATCGTCATCACGGTCTTCGTGCTCAATGTGCACCACCGCTCG
CCACGCACACACACGATGCCCGCCTGGGTGCGTAGAGTCTTCCTGGACATCGTGCCTCGCCTC
CTCTTCATGAAGCGCCCCTCTGTGGTCAAAGACAACTGCCGGAGACTTATTGAGTCCATGCAC
AAGATGGCCAACGCCCCCCGCTTCTGGCCAGAGCCTGTGGGCGAGCCCGGCATCTTGAGTGAC
ATCTGCAACCAAGGTCTGTCACCTGCCCCAACTTTCTGCAACCCCACGGACACAGCAGTCGAG
ACCCAGCCTACGTGCAGGTCACCCCCCCTTGAGGTCCCTGACTTGAAGACATCAGAGGTTGAG
AAGGCCAGTCCCTGTCCATCGCCTGGCTCCTGTCCTCCACCCAAGAGCAGCAGTGGGGCTCCA
ATGCTCATCAAAGCCAGGTCCCTGAGTGTCCAGCATGTGCCCAGCTCCCAAGAAGCAGCAGAA
GATGGCATCCGCTGCCGGTCTCGGAGTATCCAGTACTGTGTTTCCCAAGATGGAGCTGCCTCC
CTGGCTGACAGCAAGCCCACCAGCTCCCCGACCTCCCTGAAGGCCCGTCCATCCCAGCTTCCC
GTGTCAGACCAGGCCTCTCCATGCAAATGCACATGCAAGGAACCATCTCCTGTGTCCCCAGTC
ACTGTGCTCAAGGCGGGAGGCACCAAAGCACCTCCCCAACACCTGCCCCTGTCACCAGCCCTG
ACACGGGCAGTAGAAGGCGTCCAGTACATTGCAGACCACCTCAAGGCAGAAGACACTGACTTC
TCGGTGAAGGAGGACTGGAAATACGTGGCCATGGTCATTGACCGAATCTTCCTCTGGATGTTC
ATCATTGTCTGCCTTCTGGGCACTGTGGGACTCTTCCTGCCTCCCTGGCTGGCTGCTTGCTGA

The base sequence (SEQ ID No. 26) of the mutated gene cDNA (S286LPB) with the probe (SEQ ID No. 23) inserted into the mutated cDNA (S286L) (i.e., S284L in humans) of the rat *Chrna4* subunit gene is indicated as below. It is to be noted herein that the mutation of c.856T>C and c.857C>T causes the bases TCT at positions 856-858 to be mutated into the mutated CTT as enclosed by circle, and the probe portion (118 bp) is indicated as underlined at positions 104-221.

### (SEQ ID No. 26)

ATGGCCAATTCGGGCACCGGGGCGCCGCCGCTGCTGCTACTGCCGCTGCTGCTGCTCCTA
GGGACCGGCCTCTTGCCTGCTAGCAGCCACATAGAGACCCGGGCTCACGCCGAAGAACGCCTG
CTCAAAAGGCTGTTTTCTGGCTATAATAAATGGTCCCGCCCCGTGGCTAACATTTCCGACGTC
GTGCTGGTGCGGTTCGGATTATCTATCGCTCAGCTCATTGACGTGGACGAGAAGAACCAGATG
ATGACAACCAACGTGTGGGTGAAGCAGGAGTGGCACGACTACAAGCTGCGCTGGGACCCTGGT
GACTACGAGAATGTCACCTCCATCCGCATCCCCTCTGAACTCATCTGGAGGCCTGACATCGTC
CTCTACAACAATGCGGATGGAGACTTTGCAGTCACCCACCTGACCAAGGCCCACCTGTTCTAT
GACGGAAGGGTGCAGTGGACACCCCCAGCCATCTATAAGAGCTCCTGCAGCATCGACGTCACC
TTCTTCCCCTTTGACCAGCAGAACTGTACCATGAAGTTTGGATCCTGGACCTACGACAAGGCC
AAGATTGACTTAGTGAGCATGCATAGCCGTGTGGACCAACTGGACTTCTGGGAAAGTGGGGAG
TGGGTCATCGTGGATGCTGTGGGCACCTACAACACCAGGAAGTACGAGTGCTGTGCCGAGATC
TATCCTGACATCACCTATGCCTTCATCATCCGACGGCTGCCGCTATTCTACACCATCAACCTC
ATCATCCCGTGCCTGCTCATCTCCTGTCTCACCGTGCTGGTCTTCTATCTGCCTTCAGAGTGT
GGCGAGAAGGTCACACTGTGCATCTCGGTGCTGCTTCTCACCGTCTTCCTGCTGCTCATC
ACCGAGATCATCCCGTCCACCTCGCTGGTCATCCCGCTCATCGGCGAGTACCTCCTCTTCACC
ATGATCTTCGTCACCCTCTCCATCGTCATCACGGTCTTCGTGCTCAATGTGCACCACCGCTCG
CCACGCACACACACGATGCCCGCCTGGGTGCGTAGAGTCTTCCTGGACATCGTGCCTCGCCTC
CTCTTCATGAAGCGCCCCTCTGTGGTCAAAGACAACTGCCGGAGACTTATTGAGTCCATGCAC
AAGATGGCCAACGCCCCCCGCTTCTGGCCAGAGCCTGTGGGCGAGCCCGGCATCTTGAGTGAC
ATCTGCAACCAAGGTCTGTCACCTGCCCCAACTTTCTGCAACCCCACGGACACAGCAGTCGAG
ACCCAGCCTACGTGCAGGTCACCCCCCCTTGAGGTCCCTGACTTGAAGACATCAGAGGTTGAG
AAGGCCAGTCCCTGTCCATCGCCTGGCTCCTGTCCTCCACCCAAGAGCAGCAGTGGGGCTCCA
ATGCTCATCAAAGCCAGGTCCCTGAGTGTCCAGCATGTGCCCAGCTCCCAAGAAGCAGCAGAA
GATGGCATCCGCTGCCGGTCTCGGAGTATCCAGTACTGTGTTTCCCAAGATGGAGCTGCCTCC
CTGGCTGACAGCAAGCCCACCAGCTCCCCGACCTCCCTGAAGGCCCGTCCATCCCAGCTTCCC

GTGTCAGACCAGGCCTCTCCATGCAAATGCACATGCAAGGAACCATCTCCTGTGTCCCCAGTC
ACTGTGCTCAAGGCGGGAGGCACCAAAGCACCTCCCCAACACCTGCCCCTGTCACCAGCCCTG
ACACGGGCAGTAGAAGGCGTCCAGTACATTGCAGACCACCTCAAGGCAGAAGACACTGACTTC
TCGGTGAAGGAGGACTGGAAATACGTGGCCATGGTCATTGACCGAATCTTCCTCTGGATGTTC
ATCATTGTCTGCCTTCTGGGCACTGTGGGACTCTTCCTGCCTCCCTGGCTGGCTGCTTGCTGA

The base sequence (SEQ ID No. 27) of the mutated gene cDNA (S282FPB) with the probe (SEQ ID No. 23) inserted into the mutated cDNA (S79-880insL) (i.e., S284L in humans) of the rat *Chrna4* subunit gene is indicated as below. In the base sequence below, the mutation of c.845C>T and c.846G>C causes the bases TCG at positions 844-846 to the mutated TTC is indicated as enclosed by circle, and the probe portion (118 bp) at positions 104-221 is indicated as underlined. The mutation regarding human beings is represented by S280F.

### (SEQ ID No. 27)

ATGGCCAATTCGGGCACCGGGGCGCCGCCGCTGCTGCTACTGCCGCTGCTGCTGCTCCTA
GGGACCGGCCTCTTGCCTGCTAGCAGCCACATAGAGACCCGGGCTCACGCCGAAGAACGCCTG
CTCAAAAGGCTGTTTTCTGGCTATAATAAATGGTCCCGCCCCGTGGCTAACATTTCCGACGTC
GTGCTGGTGCGGTTCGGATTATCTATCGCTCAGCTCATTGACGTGGACGAGAAGAACCAGATG
ATGACAACCAACGTGTGGGTGAAGCAGGAGTGGCACGACTACAAGCTGCGCTGGGACCCTGGT
GACTACGAGAATGTCACCTCCATCCGCATCCCCTCTGAACTCATCTGGAGGCCTGACATCGTC
CTCTACAACAATGCGGATGGAGACTTTGCAGTCACCCACCTGACCAAGGCCCACCTGTTCTAT
GACGGAAGGGTGCAGTGGACACCCCCAGCCATCTATAAGAGCTCCTGCAGCATCGACGTCACC
TTCTTCCCCTTTGACCAGCAGAACTGTACCATGAAGTTTGGATCCTGGACCTACGACAAGGCC
AAGATTGACTTAGTGAGCATGCATAGCCGTGTGGACCAACTGGACTTCTGGAAAGTGGGGAG
TGGGTCATCGTGGATGCTGTGGGCACCTACAACACCAGGAAGTACGAGTGCTGTGCCGAGATC
TATCCTGACATCACCTATGCCTTCATCATCCGACGGCTGCCGCTATTCTACACCATCAACCTC
ATCATCCCGTGCCTGCTCATCTCCTGTCTCACCGTGCTGGTCTTCTATCTGCCTTCAGAGTGT
GGCGAGAAGGTCACACTGTGCATCTCGGTGCTGCTTTCTCTCACCGTCTTCCTGCTGCTGCTA
ATCACCGAGATCATCCCGTCCACCTCGCTGGTCATCCCGCTCATCGGCGAGTACCTCCTCTTC

ACCATGATCTTCGTCACCCTCTCCATCGTCATCACGGTCTTCGTGCTCAATGTGCACCACCGC
TCGCCACGCACACACACGATGCCCGCCTGGGTGCGTAGAGTCTTCCTGGACATCGTGCCTCGC
CTCCTCTTCATGAAGCGCCCCTCTGTGGTCAAAGACAACTGCCGGAGACTTATTGAGTCCATG
CACAAGATGGCCAACGCCCCCGCTTCTGGCCAGAGCCTGTGGCGAGCCCGGCATCTTGAGT
GACATCTGCAACCAAGGTCTGTCACCTGCCCCAACTTTCTGCAACCCCACGGACACAGCAGTC
GAGACCCAGCCTACGTGCAGGTCACCCCCCTTGAGGTCCCTGACTTGAAGACATCAGAGGTT
GAGAAGGCCAGTCCCTGTCCATCGCCTGGCTCCTGTCCTCCACCCAAGAGCAGCAGTGGGGCT
CCAATGCTCATCAAAGCCAGGTCCCTGAGTGTCCAGCATGTGCCCAGCTCCCAAGAAGCAGCA
GAAGATGGCATCCGCTGCCGGTCTCGGAGTATCCAGTACTGTGTTTCCCAAGATGGAGCTGCC
TCCCTGGCTGACAGCAAGCCCACCAGCTCCCCGACCTCCCTGAAGGCCCGTCCATCCCAGCTT
CCCGTGTCAGACCAGGCCTCTCCATGCAAATGCACATGCAAGGAACCATCTCCTGTGTCCCCA
GTCACTGTGCTCAAGGCGGGAGGCACCAAAGCACCTCCCCAACACCTGCCCCTGTCACCAGCC
CTGACACGGGCAGTAGAAGGCGTCCAGTACATTGCAGACCACCTCAAGGCAGAAGACACTGAC
TTCTCGGTGAAGGAGGACTGGAAATACGTGGCCATGGTCATTGACCGAATCTTCCTCTGGATG
TTCATCATTGTCTGCCTTCTGGGCACTGTGGGACTCTTCCTGCCTCCCTGGCTGGCTGCTTGC
TGA

The base sequence (SEQ ID No. 28) of the mutated gene cDNA (V286L) with a probe (SEQ ID No. 24) inserted into the mutated cDNA of the rat *Chrnb2* subunit gene is indicated as below.

### (SEQ ID No. 28)

GGCCGGGCACTCCAACTCAATGGCGCTGTTCAGCTTCAGCCTTCTTTGGCTGTGCTCAGGGGT
TTTGGGAACTGACACAGAGGAGCGGCTAGTGGAGCATCTCTTAGATCCCTCCCGCTATAACAA
GCTGATTCGTCCAGCTACTAACGGCTCTGAGCTGGTGACTGTACAGCTCATGGTATCATTGGC
TCAGCTCATTAGTGTGCACGAGCGGGAGCAGATCATGACCACCAATGTCTGGCTGACCCAGGA
GTGGGAAGATTACCGCCTCACATGGAAGCCTGAGGACTTCGACAATATGAAGAAAGTCCGGCT
CCCTTCCAAACACATCTGGCTCCCAGATGTGGTTCTATACAACAATGCTGACGGCATGTACGA
AGTCTCCTTCTATTCCAATGCTGTGGTCTCCTATGATGGCAGCATCTTTTGGCTACCACCTGC
CATCTACAAGAGTGCATGCAAGATTGAGGTGAAGCACTTCCCATTTGACCAGCAGAATTGCAC
CATGAAGTTTCGCTCATGGACCTACGACCGTACTGAGATTGACCTGGTGCTCAAAAGTGATGT

GGCCAGTCTGGATGACTTCACACCCAGCGGGGAGTGGACATCATCGCACTGCCAGGCCGACG
CAACGAGAACCCAGACGACTCCACCTATGTGGACATCACCTATGACTTCATCATTCGTCGCAA
ACCACTCTTCTACACTATCAACCTCATCATCCCCTGCGTACTCATCACCTCGCTGGCCATCCT
GGTCTTCTACCTGCCCTCAGACTGTGGTGAAAAGATGACACTTTGTATTTCTGTGCTGCTAGC
ACTCACGGTGTTCCTGCTGCTCATCTCCAAGATTCTGCCTCCCACCTCCCTCGATGTACCGCT
GGTGGGCAAGTACCTCATGTTTACCATGGTGCTAGTCACCTTCTCCATCGTCACCAGCGTGTG
TGTGCTCAATGTGCACCACCGCTCGCCTACCACGCACACCATGGCCCCCTGGGTCAAGGTGGT
CTTCCTGGAGAAGCTGCCCACCCTGCTCTTCCTGCAGCAGCCACGCCACCGCTGTGCACGTCA
GCGTCTGCGCTTGAGGAGGCGCCAGCGAGAGCGTGAGGGCGCAGGCGCGCTTTTCTTCCGTGA
AGGTCCTGCGGCTGACCCATGTACCTGCTTTGTCAACCCCGCCTCCGTCCAAGGACTCGCCGG
CGCCTTTAGGGCCGAACCTACCGCCGCTGGCCCGGGGCGCTCTGTGGGGCCATGCAGCTGTGG
CCTCCGGGAAGCAGTGGATGGCGTACGCTTCATTGCGGACCACATGCGAAGTGAGGATGATGA
CCAGAGTGTGAGGGAGGACTGGAAATACGTTGCCATGGTGATCGACCGCCTGTTCCTGTGGAT
CTTTGTCTTTGTCTGTGTCTTTGGGACCGTCGGCATGTTCCTGCAGCCTCTCTTCCAGAACTA
CACTGCCACTACCTTCCTCCACCCTGACCACTCAGCTCCCAGCTCCAAGTGA

The base sequence (SEQ ID No. 29) of the mutated gene cDNA (V286M) with a probe (SEQ ID No. 24) inserted into the mutated cDNA of the rat *Chrnb2* subunit gene is indicated as below.

### (SEQ ID No. 29)

TGGCCGGGCACTCCAACTCAATGGCGCTGTTCAGCTTCAGCCTTCTTTGGCTGTGCTCAGGGG
TTTTGGGAACTGACACAGAGGAGCGGCTAGTGGAGCATCTCTTAGATCCTCCCGCTATAACA
AGCTGATTCGTCCAGCTACTAACGGCTCTGAGCTGGTGACTGTACAGCTCATGGTATCATTGG
CTCAGCTCATTAGTGTGCACGAGCGGGAGCAGATCATGACCACCAATGTCTGGCTGACCCAGG
AGTGGGAAGATTACCGCCTCACATGGAAGCCTGAGGACTTCGACAATATGAAGAAAGTCCGGC
TCCCTTCCAAACACATCTGGCTCCCAGATGTGGTTCTATACAACAATGCTGACGGCATGTACG
AAGTCTCCTTCTATTCCAATGCTGTGGTCTCCTATGATGGCAGCATCTTTTGGCTACCACCTG
CCATCTACAAGAGTGCATGCAAGATTGAGGTGAAGCACTTCCCATTTGACCAGCAGAATTGCA
CCATGAAGTTTCGCTCATGGACCTACGACCGTACTGAGATTGACCTGGTGCTCAAAAGTGATG
TGGCCAGTCTGGATGACTTCACACCCAGCGGGGAGTGGGACATCATCGCACTGCCAGGCCGAC

GCAACGAGAACCCAGACGACTCCACCTATGTGGACATCACCTATGACTTCATCATTCGTCGCA
AACCACTCTTCTACACTATCAACCTCATCATCCCCTGCGTACTCATCACCTCGCTGGCCATCC
TGGTCTTCTACCTGCCCTCAGACTGTGGTGAAAAGATGACACTTTGTATTTCTGTGCTGCTAG
CACTCACGGTGTTCCTGCTGCTCATCTCCAAGATTATGCCTCCCACCTCCCTCGATGTACCGC
TGGTGGGCAAGTACCTCATGTTTACCATGGTGCTAGTCACCTTCTCCATCGTCACCAGCGTGT
GTGTGCTCAATGTGCACCACCGCTCGCCTACCACGCACACCATGGCCCCCTGGGTCAAGGTGG
TCTTCCTGGAGAAGCTGCCCACCCTGCTCTTCCTGCAGCAGCCACGCCACCGCTGTGCACGTC
AGCGTCTGCGCTTGAGGAGGCGCCAGCGAGAGCGTGAGGGCGCAGGCGCGCTTTTCTTCCGTG
AAGGTCCTGCGGCTGACCCATGTACCTGCTTTGTCAACCCCGCCTCCGTCCAAGGACTCGCCG
GCGCCTTTAGGGCCGAACCTACCGCCGCTGGCCCGGGGCGCTCTGTGGGGCCATGCAGCTGTG
GCCTCCGGGAAGCAGTGGATGGCGTACGCTTCATTGCGGACCACATGCGAAGTGAGGATGATG
ACCAGAGTGTGAGGGAGGACTGGAAATACGTTGCCATGGTGATCGACCGCCTGTTCCTGTGGA
TCTTTGTCTTTGTCTGTGTCTTTGGGACCGTCGGCATGTTCCTGCAGCCTCTCTTCCAGAACT
ACACTGCCACTACCTTCCTCCACCCTGACCACTCAGCTCCCAGCTCCAAGTGA

By using the rat *Chrna4* or *Chrnb2* cDNA with the mutation and the probe produced by the above techniques, the model non-human mammalian animals for human epilepsy according to the present invention may be produced by non-human mammalian animals-producing methods known per se in the art. Such methods may include, for example, a transgenic animals-producing method involved in introduction of a gene of interest into an animal individual, a method of genetic replacement animals involved in replacement of a target gene by a gene of interest, a clone producing method involved in using a nucleus of a cell produced by modifying a gene of interest, and so on.

In this description, there is described the transgenic animals-producing method for producing transgenic animals such as rats, for example, by introducing the cDNA of the mutated gene as an objective gene into a fertilized egg of the individual animal.

First, an expression vector is formed, which includes a DNA fragment encoding a mutated gene having the above mutation in the gene homologous to the α4 subunit *(CHRNA4)* or the β2 subunit (*CHRNB2*) of the neuronal nicotinic acetylcholinergic receptor gene associated with human autosomal dominant nocturnal frontal lobe epilepsy.

More specifically, the expression vector can be constructed by isolating the DNA fragment containing the α4 subunit (*Chrna4*) or the β2 subunit *(Chrnb2)* of the neuronal nicotinic acetylcholinergic receptor gene associated with human autosomal dominant nocturnal frontal lobe epilepsy from the non-human mammalian animal and inserting an exogenous gene, etc. into the DNA fragment. Such an exogenous gene may include, for example, preferably a marker gene, particularly preferably an antibiotic-resistant gene such as a neomycin-resistant gene and so on. In the event where such an antibiotic-resistant gene is inserted, a homologously recombinant cell line can be selected simply by incubating the cell line in culture medium containing the corresponding antibiotic. Further, in order to achieve a more efficient selection, it is preferred to connect a thymidine kinase gene or the like to the expression vector.
This can exclude the cell lines with no homologous recombination. Moreover, it is possible to exclude the non-homologously recombinant cell lines by connecting diphteria toxin A fragment (DT-A) gene or the like to the expression vector. Such vectors may include, for example, pCI-neo, pMCIneo, pXT1, pSG5, pcDNA3.neo, pLITMUS28, pcDNAIamp,pcDNA3, and so on.

The above DNA fragment may be preferably inserted in the non-human mammalian animal by using the expression vector as an insertion gene to which the DNA fragment is connected at a location downstream of an appropriate promoter. As a promoter, there may be used any promoter that can initiate transcription in the cells of the non-human mammalian animal to which a coding DNA of the receptor subunit gene is introduced. The promoter is not limited to a particular one and may include, for example, a gene derived from a virus such as simian virus 40, polyomavirus, adenovirus 2, human cytomegalovirus, retrovirus, or the like.

The expression vector to be used for the present invention may preferably possess a poly-A additional signal necessary for polyadenylation at the 3'-terminus of mRNA at a position located downstream of the DNA encoding the mutated gene of the α4 subunit (*Chrna2*) or the β2 subunit (*Chrnb2*) of the neuronal nicotinic acetylcholinergic receptor gene of a non-human mammalian animal. As such a poly-A additional signal, there may be used, for example, a poly-A additional signal of the late gene or the early gene of SV40 contained in each gene derived from the above virus, etc. Moreover, in order to more highly express the gene of interest, the expression vector may preferably contain a splicing signal, an enhancer region, or a portion of intron of each gene, ligated at a 5'-downstream location of the promoter region or between the promoter region and a translation region or at a 3'-downstream location of the translation region.

In en embodiment according to the present invention, for instance, the mutated rat *Chrna4* or *Chrnb2* cDNA with the above mutation and the probe introduced thereinto is transferred to the expression vector using restriction enzymes *Xho I* and *NotI* and then cleaved with restriction enzymes *SnaBI* and *NaeI.* For instance, on one hand, in the event where the mutated rat *Chrna4* cDNA is to be transferred to an expression vector having a PDGF promoter derived from a pCI-neo vector, it can be transferred with restriction enzymes *XhoI* and *Not I* and then cleaved with restriction enzymes SnaBI and *Nae I.* Further, in the event where the mutated rat *Chrnb2* cDNA is to be transferred thereto, it can be transferred with restriction enzymes *Xho I* and *Sal I* and then cleaved with restriction enzymes *SnaBI* and *DraIII.* This allows a fragment consisting of the PDGF promoter, the mutated rat *Chrna4* or *Chrnb2* cDNA and the poly-A portion can be isolated and purified as a DNA construct by gel processing.

In an embodiment according to the present invention, for instance, the DNA construct isolated and purified as described above is then introduced into differentially totipotent cells of the non-human mammalian animal leading to the production of the non-human mammalian animal as a model animal of interest. Examples of the totipotent cells may include, for example, fertilized eggs, early embryos, embryonic stem cells, and so on. The fertilized eggs to be used for introduction of the DNA construct may be obtained, for example, by intraperitoneally administering the animal with an ovulatory drug such as gonadotropic hormone (PMS), etc., inducing superovulation in female animals and collecting fertilized eggs usable for introduction of the DNA construct. A pseudopregnant female animal with the fertilized eggs is crossed with a male mouse castrated by vasoligation, etc. and the oviduct of the pseudopregnant female animal was removed to collect the eggs by retrieving them under a microscope.

To the resulting fertilized eggs is then introduced the DNA construct. Although various methods are known for introducing the DNA construct to the fertilized eggs, microinjection is preferred in order to promote a production efficiency of transgenic animals and a transmission efficiency of the inserted gene to the subsequent generations. The fertilized egg with the mutated gene injected thereinto is transplanted to the oviduct of a foster mother resulting in a delivery of offspring. After delivery, the DNA is then extracted from the somatic cells of a portion of the body (e.g., the tail tip) to confirm the presence of the transferred gene by southern blotting or PCR in order to select the transgenic animals of interest from the delivered offspring individuals, which possess the introduced gene in the genome of the somatic cells.

The individual (i.e., a heterozygote) with the presence of the inserted gene confirmed as above is determined as a founder (F0). As the introduced gene is transmitted by 50% to its offspring (F1). By crossing the F1 female individual with the F1 male individual, individuals (F2) having the gene in both of the diploid chromosomes can be produced.

In contrast, the transgenic method for forming the model non-human mammalian animals for human epilepsy according to the present invention can introduce the mutated gene encoding a mutated *Chrna4* or *Chrnb2* gene at an optional position of the chromosomal DNA in such a state that the endogenous gene (*Chrna4* or *Chrnb2* gene) is present in a normal state. Therefore, the resulting human epilepsy model animals according to the present invention can simultaneously produce normal *Chrna4* or *Chrnb2* gene as well as mutated *Chrna4* or *Chrnb2* gene, respectively. As the neuronal nicotinic acetycholinergic receptor is a protein that functions as an ion channel (consisting of two α subunits and three β subunits), the channel function may be altered if either of the subunits would be mutated. Therefore, as in the present invention, it is considered to be appropriate that the human epilepsy model animals demonstrate epileptic seizures of a human type upon simultaneous expression of both the normal subunit and the mutated subunit.

The model non-human mammalian animals for human epilepsy according to the present invention possess supeior properties of spontaneously developing epileptic seizures during sleep as in the case of human autosomal dominant nocturnal frontal lobe epilepsy as will be described under Examples below.

The individuals with the gene of interest incorporated in the chromosomes of all the cells may be selected usually by preparing DNA from a portion of tissues such as blood tisues, epithelial tissues and so on and subjecting the resulting DNA to DNA sequencing to confirm the presence of the introduced gene at a DNA level. The selection of the recombinant indivisual by the DNA sequencing as above, however, requires laborious work and a long labor time as well as a large amount of expenses.

In other words, in the event where disease model animals with genetic abnormality are produced, a homologous recombinant is required to be determined in an accurate way. At this end, the conventional method for producing disease model animals requires sequencing of a portion of the gene of an indivisual and expressing mRNA of the introduced gene. It is not easy, however, to express mRNA of the introduced gene by RT-PCR, in situ hybridization, and so on.

Therefore, the present invention is devised so as to easily distinguishing the recombinants by introducing the mutated gene as described above and replacing a portion of the mutated gene by a probe. Moreover, the present invention enables an easy distinction of the recombinants by causing a new restriction site of a restriction enzyme to appear by the mutation as above. It is to be understood herein that this approach is not restricted to specific embodiments as will be described below and it can be commonly applied to every mutation regardless of types of mutations and kinds of restriction enzymes, etc. In addition, the present invention does not particularly restrict the type of the mutation for introduction into a specific site of the cDNA clone to a specific one.

More specifically, in order to allow for easy selection of such recombinant individuals, the present invention is provided in such a manner that a new restriction site of the restriction enzyme is caused to appear by introducing the mutation such as, for example, S280L, S284L, S291-292L, V287L, V287M, etc., into the DNA fragment encoding the α4 subunit (*Chrna4*) or the β2 subunit (*Chrnb2*) of the neuronal nicotinic acetylcholinergic receptor gene of the non-human mammalian animal. The appearance of the new restriction site of the restriction enzyme can easily distinguishe the recombinants upon formation of the recombinants.

In an embodiment of the present invention, the base sequence of a portion of the mutated cDNA is replaced by a probe with a base sequence that is substantially thoroughly different therefrom yet encodes an amino acid sequence identical thereto, and the probe is introduced at the site identical to the site of the original base sequence. Therefore, the recombinant individuals can be easily distinguished using the restriction enzymes for the introduced probe. Moreover, the mRNA expressed from the recombinant gene from the original rat *Chrna4* mRNA or *Chrnb2* mRNA can be detected easily by techniques, such as RT-PCR or in situ hybridization, etc.

Further, for instance, a restriction site of restriction enzyme *Hinf If* is caused to occur by introduction of the mutation (c.856G>C) or (c.856G>A) to the homologous gene *Chrnb2* of the β2 subunit *(CHRNB2)* of the neuronal nicotinic acetylcholinergic receptor gene. Therefore, the region containing this restriction site can be identified using the restriction enzyme *Hinf If* by PCR-RFLP (restriction fragment length polymorphism), etc. This indicates that, on one hand, an individual with the given homologous recombination of interes possesses the restriction site of the restriction enzyme *Hinf If* so that the restriction site involved is cleaved by the restriction enzyme *Hinf If* and that, on the other hand, an individual with no given homologous recombination does not possess the restriction site of the restriction enzyme *Hinf If* so that no cleavage is caused to occur by the restriction enzyme *Hinf If.* Therefore, the present invention allows a ready distinction of the recombinants by the mutation of the homologous gene *Chrna4* or *Chrnb2* of the α4 subunit *CHRNA4* or the β4 subunit *CHRNB2,* respectively, of the neuronal nicotinic acetylcholinergic receptor gene so as to cause an appearance of a restriction site of the corresponding restriction enzyme.

It is noted herein that the PCR-RFLP method to be used herein is a method well known per se in the art and this method enables a detection of a presence or absence of a restriction site of a restriction enzyme by amplifying the involved portions by PCR, digestion of the amplified product with a given restriction enzyme such as *True9I* or *Hinf If,* and determination of the digested DNA fragment size using electrophoresis, etc.

The present invention will be described more in detail by way of examples. It is to be understood that the present invention is not interpreted as being restricted in any respect by the following examples and that the following examples are described solely for illustrative purposes to explain the invention more specifically.

### EXAMPLE 1

First, PCR was performed using a primer set consisting of two primers designed based on a known cDNA sequence of rat Chrna4 using Rat Brain QUICK-Clone cDNA (CLONTECH; Mountain View, CA) as a template, namely,
a forward primer (BN-Rat *CHRNA*4 cDNA-F):
AGATCTCGCGAAGCTTCACCATGGCCAATTCGGGCACCGG (40 mer; SEQ ID NO: 3), and
a reverse primer (Rat *CHRNA*4 cDNA-BX-R):
   AGATCTAGATCAGCAAGCAGCCAGCCAGGGAGGCAGGA (38 mer; SEQ ID
   NO: 4).
The PCR product was purified and subcloned into a pCRII-TOPO vector (Invitorogen; Carlsbad, CA). The resulting clone was sequenced to confirm the base sequence of the *Chrna*4 cDNA. The rat base sequence information is available under accession number L31620 (NCBI).

Mutation was introduced into the clone using QuikChange Site-Directed Mutagenesis Kit (STRATAGENE; La Jolla, CA). First, the mutation was performed by mutating cytosine C at position 845 of the cDNA base sequence to thymine T and guanine G at position 846 thereof to cytosine C, respectively, using: r845T846C-sen: CACACTGTGCATCTTCGTGCTGCTTTCTC (29 mer; SEQ ID NO: 7); and r845T846C-ant:
GAGAAAGCAGCACGAAGATGCACAGTGTG (29 mer; SEQ ID NO: 8). The base sequence of the mutated cDNA is as shown in SEQ ID NO: 9. This allows a mutation of amino acid residue Ser to amino acid residue Phe mutation at position p.282 homologous to the α4 subunit of the human neuronal nicotinic acetylcholinergic receptor gene.

A probe was prepared, which consists of a 118 bp nucleotide (SEQ ID NO:23) having a base sequence thoroughly different from its original base sequence yet coding for the same amino acid sequence at positions c.104-c.221 of the mutated cDNA.

### (SEQ ID NO:23)

GGGCTCACGCCGAAGAACGCCTGCTCAAAAGGCTGTTTTCTGGCTATAATAAATGGTCCCGCC

CCGTGGCTAACATTTCCGACGTCGTGCTGGTGCGGTTCGGATTATCTATCGCTCA

After hybridization of the mutated cDNA with the introduced probe, the hybridized cDNA was inserted into a pCRII-TOPO vector at the given restriction site of the *Chrna*4 cDNA inserted in the vector using the restriction enzyme sites *SmaI* and *Bpu1102I.* The insert was sequenced in each step, and the base sequence was confirmed.

The rat *Chrna*4 cDNA with the mutation (S286L) was transferred to an expression vector with a PDGF promoter derived from a pCI-neo vector using the restriction enzyme sites *XhoI* and *NotI*, and then cut with restriction enzymes *SnaBI* and *NaeI.* A fragment including the PDGF promoter, the rat *Chrna*4 cDNA and a poly A portion was isolated by gel processing and purified. The isolated and purified DNA was microinjected into fertilized eggs, and over 200 eggs in good condition were transplanted into the oviduct of recipient females. The animals were allowed to give natural birth to recombinants.

### EXAMPLE 2

In substantially the same manner as in Example 1, the mutation was performed by mutating thymine T to cytosine C at position 856 of the cDNA base sequence and cytosine C to thymine T at position 857 thereof, respectively, using: r856C857T-sen:
CGGTGCTGCTTCTTCTCACCGTCTTCCTG (29 mer; SEQ ID NO: 10), and
r856C857T-ant:
   CAGGAAGACGGTGAGAAGAAGCAGCACCG (29 mer; SEQ ID NO: 11).
      The base sequence of the resulting mutated cDNA is as shown in SEQ ID NO: 12. This allows a mutation of amino acid residue Ser to amino acid residue Leu at position p.286 homologous to the α4 subunit of the human neuronal nicotinic acetylcholinergic receptor gene.
      A probe was prepared in substantially the same manner as in Example, which consists of a 118 bp nucleotide (SEQ ID NO:23) located at positions c.104-c.221 of the mutated cDNA.

The recombinants were created in substantially the same manner as in Example 1 using the mutated cDNA with the probe introduced therein as above.

### EXAMPLE 3

In substantially the same manner as in Example 1, GCT was inserted between positions 878 and 879 of the cDNA base sequence, using the sense primer:
r*CHRNA*-878insGCT-sen:
   GTCTTCCTGCTGCTGCTCATCACCGAGATC (30 mer; SEQ ID NO: 13),
   and the antisense primer:
r*CHRNA*-878insGCT-ant:
   GATCTCGGTGATGAGCAGCAGCAGGAAGAC (30 mer; SEQ ID NO: 14).
The base sequence of the resulting mutated cDNA is as shown in SEQ ID NO: 15. This mutation allows an insertopn of amino acid residue Leu between the amino acid residues Leu and Ile at positions p.293 and p.294, respectively, homologous to the α4 subunit of the human neuronal nicotinic acetylcholinergic receptor gene.

The mutated cDNA was then used to create recombinants in substantially the same manner as in Example 1.

### EXAMPLE 4

The mutations c.856G>C and c.856G>A were introduced into the rat *Chrnb*2 isolated by PCR cloning, using a mutation introducing method. Specifically, PCR was performed using a primer set consisting of two primers designed based on a known cDNA sequence of rat Chrnb2 using Rat Brain QUICK-Clone cDNA (CLONTECH; Mountain View, CA) as a template, namely, BN-Rat *CHRNB2* cDNA-F:
AGATCTCGCGACATGGCCGGGCACTCCAACTCAATGGCGCT (41 mer; SEQ ID NO: 5), and
Rat *CHRNB*2 cDNA-CB-R: ATCGATGGATCCTCACTTGGAGCTGGGAGCTGAGTGGTCA (40 mer; SEQ ID NO: 6).
The PCR product was purified and subcloned into a pCRII-TOPO vector (Invitorogen; Carlsbad, CA). The resulting clone was sequenced to confirm the base sequence of the *Chrnb2* cDNA. The rat base sequence information is available under accession number NM_019297 (NCBI).

The mutation was introduced into the clone using QuikChange Site-Directed Mutagenesis Kit (STRATAGENE, La Jolla, CA). First, guanine G was mutated to cytosine C at position 856 of the cDNA base sequence using:
Rat *CHRNB*2 m286V/M-F: CTCATCTCCAAGATTATGCCTCCCACCTCC (30 mer; SEQ ID NO: 17), and
Rat *CHRNB*2 m286V/M-R: GGAGGTGGGAGGCATAATCTTGGAGATGAG (30 mer; SEQ ID NO: 18).
This mutation results in a mutation of amino acid residue Val to amino acid residue Leu at position p.286 homologous to the β2 subunit of the human neuronal nicotinic acetylcholinergic receptor gene (β is used to comply with the on-line application regulations set by JPO).

The introduction of the mutation c.856G>C was arranged so as to cause a restriction enzyme site *Hinf If* to appear, thereby enabling an easy identification of the recombinants upon creating recombinants.

A probe (SEQ ID NO: 24) having a base sequence different from the original base sequence yet encoding the same amino acid sequence was introduced at the site of the mutated cDNA located from c.1171 to c.1232.

### (SEQ ID NO:24)

AACCCCGCCTCCGTCCAAGGACTCGCCGGCGCCTTTAGGGCCGAACCTACCGCCGCTGGCCC

After the 62-bp nucleotide was prepared and hybridized, it was inserted into a pCRII-TOPO vector using the restriction enzyme sites *Hinc II* and *Sma I* of the *Chrnb*2 cDNA inserted in the vector. The insert was sequenced in each step, and the base sequence was confirmed.

The two types of rat *Chrnb*2 cDNA with the mutation and the probe were transferred to an expression vector with a PDGF promoter derived from a pCI-neo vector, using the restriction enzyme sites *Xho I* and *Sal I*, and then cut with restriction enzymes *SnaBI* and *DraIII.* The fragment including the PDGF promoter, the rat *Chrnb*2 cDNA and a poly A portion was isolated by gel processing and purified. The isolated DNA was microinjected into fertilized eggs, and over 200 eggs in good condition were transplanted into the oviduct of recipient females. The animals were allowed to give natural birth to recombinants.

### EXAMPLE 5

In substantially the same manner as in Example 4, guanine G was mutated to adenine A mutation at position 856 of the cDNA base sequence using:
Rat *CHRNB*2 m286V/L-F: CTCATCTCCAAGATTCTGCCTCCCACCTCC (30 mer; SEQ ID NO: 20), and
Rat *CHRNB*2 m286V/L-R: GGAGGTGGGAGGCAGAATCTTGGAGATGAG (30 mer; SEQ ID NO: 21).
This mutation allows a replacement of amino acid residue Val by amino acid residue Met at position p.286 homologous to the β2 subunit of the human neuronal nicotinic acetylcholinergic receptor. The mutated cDNA was used to create recombinants in substantially the same manner as in Example 4.

### EXAMPLE 6

### Epilepsy Model Rat Electroencephalography (EEG)

The transgenic rats (*Chrnb*2 V287L), 8 to 10 weeks of age, were subjected to electroencephalography (EEG). The rats were anesthetized with nembutal (Dainippon Sumitomo Pharma Co., Ltd.), and anchored to a brain stereotaxis apparatus (SR-5, Narishige). The electrodes were placed 2.5 mm (arterial) and 1.5 mm (lateral), and -2.5 mm (arterial) and 2.5 mm (lateral) from the bregma of the rat skull. The indifferent electrode was placed on a thick portion of the rat bone in the vicinity of the nasal bone. A dental cement was used to fix these electrodes. Teflon^{®}-coated stainless steel electrodes for electroencephalography were placed on the frontal lobes of the left and right cerebral hemispheres (A = 3. mm, L = 0.8 mm from the bregma), and fixed with a dental cement. The indifferent electrode was implanted on the upper part of the cerebellum portion. The electroencephalogram (EEG) was taken with a VideoOption (Kissei Comtec) under freely moving conditions in a chamber over a time period of several days. Electroencephalogram analysis was made using a SleepSign (Kissei Comtec).

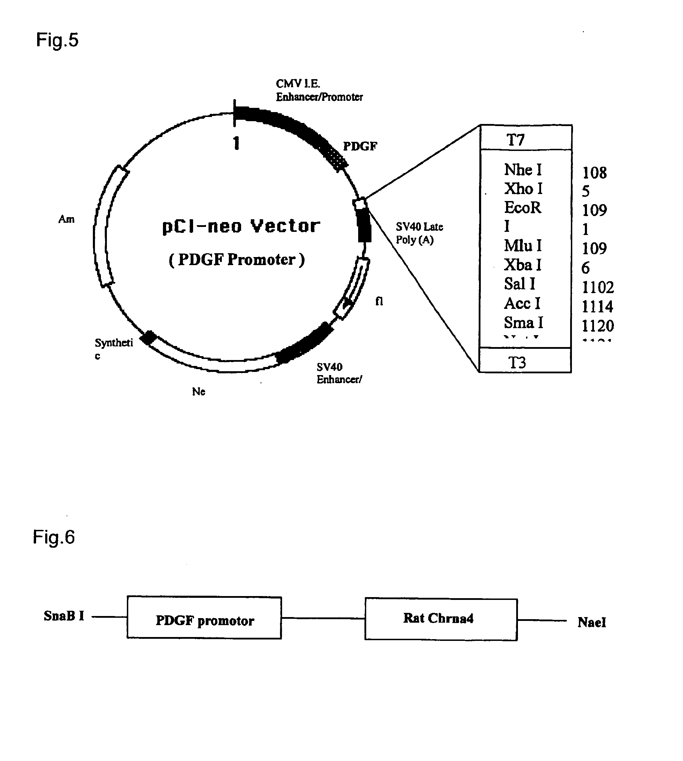

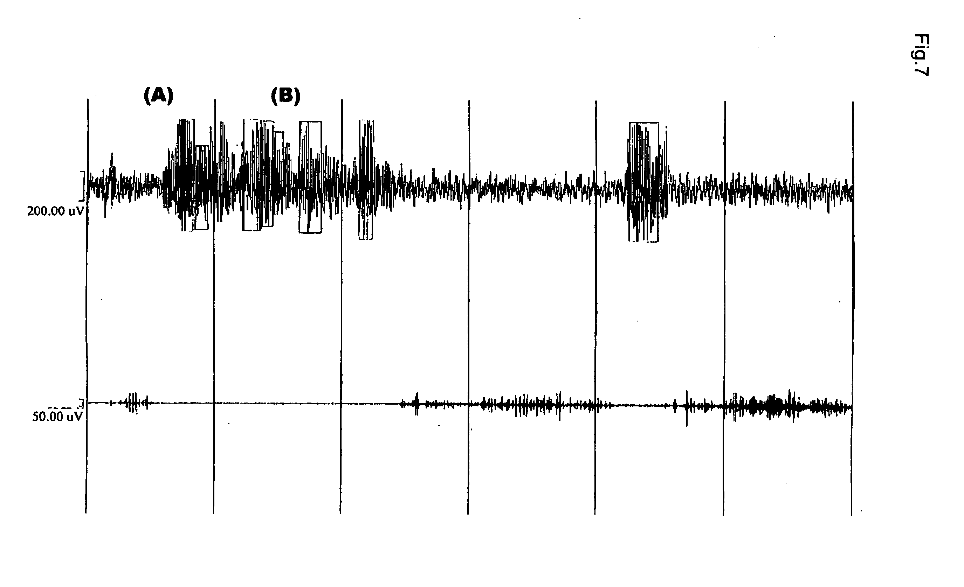

Fig. 5 represents the result of electroencephalography. In the figure, portions of abnormal brain waves are indicated by symbols A and B. Fig. 6 is a magnified view of the abnormal brain wave portion A. Fig. 7 shows the abnormal brain wave portion B. The results confirmed that the rat according to the present invention is usable as a model animal of epilepsy.

### INDUSTRIAL AVAILABILITY

A model non-human mammalian animal for human epilepsy according to the present invention has the same genetic defects as those in human epilepsy. The model animal can thus be used for the studies of epilepsy by inducing seizure attacks equivalent to human epileptic seizures. The model animal is therefore usable for the research and development of therapeutic drugs for epilepsy.

## Claims

1. A model non-human mammalian animal for human epilepsy with genetic defects identical to genetic defects in human epilepsy, **characterized in that** the model non-human mammalian animal for human epilepsy possesses a mutated gene which is produced by introducing a genetic mutation to a non-human mammalian *Chrna4* or *Chrnb2* of the α4 subunit *CHRNA4* or β2 subunit *CHRNB*2 of the neuronal nicotinic acetylcholinergic receptor gene, respectively, associated with human autosomal dominant nocturnal frontal lobe epilepsy and which includes a probe having a base sequence different from the original base sequence yet coding for the same amino sequence.

2. The model non-human mammalian animal for human epilepsy as claimed in claim 1, wherein the mutation in the mutated gene causes an appearance of a new restriction site for a restriction enzyme.

3. The model non-human mammalian animal for human epilepsy as claimed in claim 1 or 2, wherein said *Chrna4* is mutated to a base sequence of SEQ ID NO. 9 in which base C (cytosine) at position 845 of cDNA thereof is mutated to base T (thymine) (c.845C>T) and base G (guanine) at position 846 thereof is mutated to base C (cytosine) (c.846G>C).

4. The model non-human mammalian animal for human epilepsy as claimed in claim 1, 2 or 3, wherein amino acid residue Ser homologous to the acid residue at position p.282 of the α4 subunit *CHRNA4* is replaced by amino acid residue Phe by introducing a mutation of c.845T>C and/or c.846G>A into cDNA of the said Chrna4 by said mutation.

5. The model non-human mammalian animal for human epilepsy as claimed in claim 1 or 2, wherein said *Chrna4* is mutated to a base sequence of SEQ ID NO. 12 in which base T (thymine) at position 856 of cDNA thereof is mutated to base C (cytosine) (c.856T>C) and/or base C (cytosine) at position 857 thereof is mutated to base T (thymine) (c.857C>T).

6. The model non-human mammalian animal for human epilepsy as claimed in claim 1, 2 or 5, wherein amino acid residue Ser homologous to the acid residue at position p.286 of the α4 subunit *CHRNA4* is replaced by amino acid residue Leu by introducing a mutation of c.856T>C and c.857C>T into Chrna4 of a non-human mammalian animal by said mutation.

7. The model non-human mammalian animal for human epilepsy as claimed in claim 1 or 2, wherein said gene created by mutating c.878-879insGCT or c.879-880insTTA in the Chrna4 is represented by SEQ ID NO: 15 or 16, respectively.

8. The model non-human mammalian animal for human epilepsy as claimed in claim 1, 2 or 7, wherein amino acid residue Leu is inserted between amino acid residue Leu homologous to the amino acid residue at position p.293 of the α 4 subunit *CHRNA4* and amino acid residue Ile homologous to the amino acid residue at position p.294 by mutation of c.878-879insGCT or c.879-880insTTA in said *Chrna4.*

9. The model non-human mammalian animal for human epilepsy as claimed in claim 1 or 2, wherein said *Chrnb2* is mutated to a base sequence of SEQ ID NO. 19 or 22 in which base G (guanine) at position 856 of cDNA thereof is mutated to base C (cytosine) (c.856G>C) or to base A (adenine) (c.856G>A), respectively.

10. The model non-human mammalian animal for human epilepsy as claimed in claim 1, 2 or 9, wherein amino acid residue Val homologous to p.286 of the β2 subunit *CHRNB2* is mutated to amino acid residue Leu or Met by mutation of c.856G>C or c.856G>A to the Chrnb2, respectively.

11. The model non-human mammalian animal for human epilepsy as claimed in any one of claims 1 to 10, wherein the probe has a base sequence represented by SEQ ID NO: 23 or 24.

12. A method for the production of a model non-human mammalian animal for human epilepsy as claimed in any one claim of claims 1 to 11, the method comprising:
preparing a mutated gene by inserting a genetic mutation in a *Chrna4* or *Chrnb2* cDNA of a non-human mammalian animal, said genetic mutation relating to genetic defects in α4 subunit *CHRNA4* or β2 subunit *CHRNB2* of neuronal nicotinic acetylcholinergic receptor gene associated with human autosomal dominant nocturnal frontal lobe epilepsy, and by replacing a part of the base sequence of the cDNA with a probe that has a base sequence different from said part of the cDNA base sequence yet codes for the same amino sequence;
transferring the mutated gene into an expression vector; and
transplanting the mutated gene in the expression vector into a recipient female via a fertilized egg to produce a recombinant non-human mammalian animal.

13. The method as claimed in claim 12, wherein the mutated gene is provided with a new restriction site for a restriction enzyme by mutation.

14. The method as claimed in claim 12 or 13, wherein:
base C (cytosine) at position 845 of *Chrna4* cDNA is mutated to base T (thymine) (c.845 C>T) and/or base G (guanine) at position 846 thereof is mutated to base C (cytosine) (c.846G>C); or
base T (thymine) at position 856 thereof is mutated to base C (cytosine) (c.856T>C) and base C (cytosine) at position 857 thereof is mutated to base T (thymine) (c.857C>T); and
a codon GCT (c.879-880insGCT) is inserted between positions 878 and 879 or a codon TTA (c.879-880insTTA) is inserted between position 879 and 880.

15. The method as claimed in claim 12, 13 or 14, wherein amino acid residue Ser at position p.282 homologous to α4 subunit *CHRNA4* is mutated to amino acid residue Phe or amino acid residue Ser at position p.284 thereof is mutated to amino acid residue Leu or amino acid residue Leu is inserted between amino acid residue Leu at position 293 thereof and amino acid residue Ile at position p.294 thereof, respectively, by mutation of c.845 C>T and c.846G>C or c.856T>C and c.857C>T or c.878-879insGCT or c.879-880insTTA in said *Chrna4* cDNA.

16. The method as claimed in claim 12 or 13, wherein base G (guanine) at position p.856 of the *Chrnb2* cDNA is mutated to base C (cytosine) (c.856G>C) or to base A (adenine) (c.856G>A).

17. The method as claimed in claim 12, 13 or 16, wherein amino acid residue Val homologous to the β2 subunit *CHRNB2* by mutation by insertion of c.856G>C or c.856G>A into the *Chrnb2* is replaced by amino acid residue Leu or Met, respectively.

18. A mutated gene comprising one or more mutations inserted in a base sequence of a gene and a part of the base sequence of the gene replaced by a probe that has a base sequence different from said part of the base sequence yet codes for the same amino acid sequence.

19. The mutated gene as claimed in claim 18, further comprising a new restriction site for a restriction enzyme to be caused to appear by insertion of the one or more mutations.

20. The mutated gene as claimed in claim 18 or 19, wherein the gene is homologous gene *Chrna*4 or *Chrnb*2 of a non-human mammalian animal homologous to α4 subunit *CHRNA4* or β2 subunit *CHRNB2* of neuronal nicotinic acetylcholinergic receptor gene associated with human autosomal dominant nocturnal frontal lobe epilepsy.

21. The mutated gene as claimed in claim 18, 19 or 20, wherein the mutated gene includes base T (thymine) at position 845 of the *Chrna4* cDNA to which base C (cytosine) is mutated (c.845C>T), and base C (cytosine) at position 846 thereof to which base G (guanine) is mutated (c.846G>C), and base C (cytosine) at position p.856 thereof to which base T (thymine) is mutated (c.856T>C) and base T (thymine) at position p.857 thereof to which base C (cytosine) is mutated (c.857C>T), or a codon GCT inserted between positions 878 and 879 thereof (878-879insGCT) or a codon TTA inserted between positions 879 and 880 (879-880insTTA).

22. The mutated gene as claimed in any one of claims 18 to 21, wherein the mutated gene includes amino acid residue Phe by which amino acid residue Ser at p.280 homologous to the α4 subunit *CHRNA4* is replaced, or amino acid residue Leu by which amino acid residue Ser at p.284 homologous thereto is replaced, or amino acid residue Leu inserted between amino acid Leu at position p.293 homologous thereto and amino acid residue Ile at position p.294 homologous thereto, respectively, by mutation of c.845 C>T and c.846G>C or c.856T>C and c.857C>T or c.878-879insGCT or c.879-880insTTA to Chrna4 cDNA.

23. The mutated gene as claimed in claim 18 or 19, wherein the muted gene includes base C (cystosine) or base A (adenine), respectively, to which base guanine at positon 856 of the *Chrnb2* cDNA is muted (c.856G>C) or (c.856G>A).

24. The mutated gene as claimed in claim 18, 19 or 23, wherein the mutated gene includes amino acid residue Leu or Met, respectively, by which amino acid residue Val at p.286 homologous to the β2 subunit *CHRNB*2 by introduction of c.856G>C or c.856G>A into the *Chrnb*2.

25. A method for creating the mutated gene as claimed in any one of claims 18 to 25, comprising:
introducing one or more mutations to the base sequence of the gene; and
replacing a part of the base sequence of the gene by inserting a probe that has a base sequence different from said part of the base sequence yet codes for the same amino acid sequence.

26. The method as claimed in claim 25, wherein a new restriction site for a restriction enzyme iscaused to appear by the one or more mutations.

27. The method as claimed in claim 25 or 26, wherein the gene is a gene *Chrna*4 or *Chrnb*2 of a non-human mammalian animal, respectively, homologous to α4 subunit *CHRNA4* or β2 subunit *CHRNB2* of neuronal nicotinic acetylcholinergic receptor gene associated with human autosomal dominant nocturnal frontal lobe epilepsy.

28. The method as claimed in claim 25, 26 or 27, wherein c.845C>T and c.846G>C or c.856T>C and c.857C>T or c.878-879insGCT or c.879-880insTTA are or is introduced into the cDNA of the *Chrna*4.

29. The method as claimed in any one of claims 25 to 28, wherein amino acid residue Ser at position p.282 homologous to the α4 subunit *CHRNA4* is replaced by amino acid residue Phe, amino acid residue Ser at position p.284 homologous thereto is replaced by amino acid residue Leu or amino acid residue Leu is inserted between amino acid residue Leu at position p.283 and amino acid residue Ile at position p.294 by introduction of the mutations c.845C>T and c.846G>C, the mutations c.856T>C and c.857C>T or the mutation c.879-880insGCT in the *Chrna*4 cDNA.

30. The method as claimed in claim 25 or 26, wherein the mutation c.856G>C or c.856G>A is introduced to the *Chrnb*2 cDNA.

31. The method as claimed in claim 25, 26 or 30, wherein amino acid residue Val homologous to the β2 subunit *CHRNB2* is replaced by amino acid residue Leu or Met, respectively, by introduction of the mutation c.856G>C or c.856G>A to the *Chrnb*2*.*

32. A method for identifying a homologous recombinant of the model non-human mammalian animal for human epilepsy as claimed in any one of claims 1 to 11, comprising detecting a probe with a restriction enzyme whether the probe is introduced into the homologous recombinant of the mutated gene as claimed in any one of claims 18 to 24, a probe having a base sequence which is different from a portion of a base sequence of a homologous gene *Chrna*4 or *Chrnb*2 of a non-human mammalian animal, respectively, homologous to α4 subunit *CHRNA4* or β2 subunit *CHRNB2* of human neuronal nicotinic acetylcholinergic receptor gene yet which codes for the same amino acid sequence as the part of the base sequence.

33. The method as claimed in claim 32, wherein the homologous recombinant of the human epilepsy model non-human mammalian animal is identified by detecting a restriction site for the restriction enzyme occurred in the muted gene using the restriction enzyme.


### Sequence Listing

SEQUENCE LISTING

<110> Fukuoka University
      Hirosaki University

<120> Epilepsy Model knockout Animals

<130> 68.106682

<150> PCT/JP2009/052230
<151> 2009-02-10

<150> JP2008-31002
<151> 2008-02-12

<160> 31

<170> PatentIn version 3.1

<210> 1
<211> 1890
<212> DNA
<213> Rattus norvegicus

<220>
<223> Rat Chrna4

<400> 1
atggccaatt cgggcaccgg ggcgccgccg ccgctgctgc tactgccgct gctgctgctc 60

ctagggaccg gcctcttgcc tgctagcagc cacatagaga cccgggccca tgcggaggag 120

cggctcctga agagactctt ctccggttac aacaagtggt ctcggccagt agccaatatc 180

tcagatgtgg tcctcgtccg ctttggcttg tccattgctc agctcattga cgtggacgag 240

aagaaccaga tgatgacaac caacgtgtgg gtgaagcagg agtggcacga ctacaagctg 300

cgctgggacc ctggtgacta cgagaatgtc acctccatcc gcatcccctc tgaactcatc 360

tggaggcctg acatcgtcct ctacaacaat gcggatggag actttgcagt cacccacctg 420

accaaggccc acctgttcta tgacggaagg gtgcagtgga cacccccagc catctataag 480

agctcctgca gcatcgacgt caccttcttc ccctttgacc agcagaactg taccatgaag 540

tttggatcct ggacctacga caaggccaag attgacttag tgagcatgca tagccgtgtg 600

gaccaactgg acttctggga aagtggggag tgggtcatcg tggatgctgt gggcacctac 660

aacaccagga agtacgagtg ctgtgccgag atctatcctg acatcaccta tgccttcatc 720

atccgacggc tgccgctatt ctacaccatc aacctcatca tcccgtgcct gctcatctcc 780

tgtctcaccg tgctggtctt ctatctgcct tcagagtgtg gcgagaaggt cacactgtgc 840

atctcggtgc tgctttctct caccgtcttc ctgctgctca tcaccgagat catcccgtcc 900

acctcgctgg tcatcccgct catcggcgag tacctcctct tcaccatgat cttcgtcacc 960

ctctccatcg tcatcacggt cttcgtgctc aatgtgcacc accgctcgcc acgcacacac 1020

acgatgcccg cctgggtgcg tagagtcttc ctggacatcg tgcctcgcct cctcttcatg 1080

aagcgcccct ctgtggtcaa agacaactgc cggagactta ttgagtccat gcacaagatg 1140

gccaacgccc cccgcttctg gccagagcct gtgggcgagc ccggcatctt gagtgacatc 1200

tgcaaccaag gtctgtcacc tgccccaact ttctgcaacc ccacggacac agcagtcgag 1260

acccagccta cgtgcaggtc accccccctt gaggtccctg acttgaagac atcagaggtt 1320

gagaaggcca gtccctgtcc atcgcctggc tcctgtcctc cacccaagag cagcagtggg 1380

gctccaatgc tcatcaaagc caggtccctg agtgtccagc atgtgcccag ctcccaagaa 1440

gcagcagaag atggcatccg ctgccggtct cggagtatcc agtactgtgt ttcccaagat 1500

ggagctgcct ccctggctga cagcaagccc accagctccc cgacctccct gaaggcccgt 1560

ccatcccagc ttcccgtgtc agaccaggcc tctccatgca aatgcacatg caaggaacca 1620

tctcctgtgt ccccagtcac tgtgctcaag gcgggaggca ccaaagcacc tccccaacac 1680

ctgcccctgt caccagccct gacacgggca gtagaaggcg tccagtacat tgcagaccac 1740

ctcaaggcag aagacactga cttctcggtg aaggaggact ggaaatacgt ggccatggtc 1800

attgaccgaa tcttcctctg gatgttcatc attgtctgcc ttctgggcac tgtgggactc 1860

ttcctgcctc cctggctggc tgcttgctga 1890

<210> 2
<211> 1503
<212> DNA
<213> Rattus norvegicus

<220>
<223> Rat Chrnb2

<400> 2
atggccgggc actccaactc aatggcgctg ttcagcttca gccttctttg gctgtgctca 60

ggggttttgg gaactgacac agaggagcgg ctagtggagc atctcttaga tccctcccgc 120

tataacaagc tgattcgtcc agctactaac ggctctgagc tggtgactgt acagctcatg 180

gtatcattgg ctcagctcat tagtgtgcac gagcgggagc agatcatgac caccaatgtc 240

tggctgaccc aggagtggga agattaccgc ctcacatgga agcctgagga cttcgacaat 300

atgaagaaag tccggctccc ttccaaacac atctggctcc cagatgtggt tctatacaac 360

aatgctgacg gcatgtacga agtctccttc tattccaatg ctgtggtctc ctatgatggc 420

agcatctttt ggctaccacc tgccatctac aagagtgcat gcaagattga ggtgaagcac 480

ttcccatttg accagcagaa ttgcaccatg aagtttcgct catggaccta cgaccgtact 540

gagattgacc tggtgctcaa aagtgatgtg gccagtctgg atgacttcac acccagcggg 600

gagtgggaca tcatcgcact gccaggccga cgcaacgaga acccagacga ctccacctat 660

gtggacatca cctatgactt catcattcgt cgcaaaccac tcttctacac tatcaacctc 720

atcatcccct gcgtactcat cacctcgctg gccatcctgg tcttctacct gccctcagac 780

tgtggtgaaa agatgacact ttgtatttct gtgctgctag cactcacggt gttcctgctg 840

ctcatctcca agattgtgcc tcccacctcc ctcgatgtac cgctggtggg caagtacctc 900

atgtttacca tggtgctagt caccttctcc atcgtcacca gcgtgtgtgt gctcaatgtg 960

caccaccgct cgcctaccac gcacaccatg gccccctggg tcaaggtggt cttcctggag 1020

aagctgccca ccctgctctt cctgcagcag ccacgccacc gctgtgcacg tcagcgtctg 1080

cgcttgagga ggcgccagcg agagcgtgag ggcgcaggcg cgcttttctt ccgtgaaggt 1140

cctgcggctg acccatgtac ctgctttgtc aaccctgcat cagtgcaggg cttggctggg 1200

gctttccgag ctgagcccac tgcagccggc ccggggcgct ctgtggggcc atgcagctgt 1260

ggcctccggg aagcagtgga tggcgtacgc ttcattgcgg accacatgcg aagtgaggat 1320

gatgaccaga gtgtgaggga ggactggaaa tacgttgcca tggtgatcga ccgcctgttc 1380

ctgtggatct ttgtctttgt ctgtgtcttt gggaccgtcg gcatgttcct gcagcctctc 1440

ttccagaact acactgccac taccttcctc caccctgacc actcagctcc cagctccaag 1500

tga 1503

<210> 3
<211> 40
<212> DNA
<213> Artificial

<220>
<223> forward primer Chrna4

<400> 3
agatctcgcg aagcttcacc atggccaatt cgggcaccgg 40

<210> 4
<211> 38
<212> DNA
<213> Artificial

<220>
<223> Reverse primer Chrna4

<400> 4
agatctagat cagcaagcag ccagccaggg aggcagga 38

<210> 5
<211> 41
<212> DNA
<213> Artificial

<220>
<223>  Forward primer Chrnb2

<400>  5
agatctcgcg acatggccgg gcactccaac tcaatggcgc t                          41


<210>  6
<211>  40
<212>  DNA
<213>  Artificial

<220>
<223>  Reverse primer Chrnb2

<400>  6
atcgatggat cctcacttgg agctgggagc tgagtggtca                            40


<210>  7
<211>  29
<212>  DNA
<213>  Artificial

<220>
<223>  Sense primer Chrna4 S282F

<400>  7
cacactgtgc atcttcgtgc tgctttctc                                        29


<210>  8
<211>  29
<212>  DNA
<213>  Artificial

<220>
<223>  Antisense primer Chrna4 S282F

<400>  8
gagaaagcag cacgaagatg cacagtgtg                                        29


<210>  9
<211>  1889
<212>  DNA
<213>  Ratuus norvegicus

<400>  9
atggccaatt cgggcaccgg ggcgccgccg ccgctgctgc tactgccgct gctgctgctc      60

ctagggaccg gcctcttgcc tgctagcagc cacatagaga cccggcccat gcggaggagc     120

ggctcctgaa gagactcttc tccggttaca acaagtggtc tcggccagta gccaatatct     180

cagatgtggt cctcgtccgc tttggcttgt ccattgctca gctcattgac gtggacgaga     240

agaaccagat gatgacaacc aacgtgtggg tgaagcagga gtggcacgac tacaagctgc     300

gctgggaccc tggtgactac gagaatgtca cctccatccg catcccctct gaactcatct     360

ggaggcctga catcgtcctc tacaacaatg cggatggaga ctttgcagtc acccacctga 420

ccaaggccca cctgttctat gacggaaggg tgcagtggac acccccagcc atctataaga 480

gctcctgcag catcgacgtc accttcttcc cctttgacca gcagaactgt accatgaagt 540

ttggatcctg gacctacgac aaggccaaga ttgacttagt gagcatgcat agccgtgtgg 600

accaactgga cttctgggaa agtggggagt gggtcatcgt ggatgctgtg ggcacctaca 660

acaccaggaa gtacgagtgc tgtgccgaga tctatcctga catcacctat gccttcatca 720

tccgacggct gccgctattc tacaccatca acctcatcat cccgtgcctg ctcatctcct 780

gtctcaccgt gctggtcttc tatctgcctt cagagtgtgg cgagaaggtc acactgtgca 840

tcttcgtgct gctttctctc accgtcttcc tgctgctcat caccgagatc atcccgtcca 900

cctcgctggt catcccgctc atcggcgagt acctcctctt caccatgatc ttcgtcaccc 960

tctccatcgt catcacggtc ttcgtgctca atgtgcacca ccgctcgcca cgcacacaca 1020

cgatgcccgc ctgggtgcgt agagtcttcc tggacatcgt gcctcgcctc ctcttcatga 1080

agcgcccctc tgtggtcaaa gacaactgcc ggagacttat tgagtccatg cacaagatgg 1140

ccaacgcccc ccgcttctgg ccagagcctg tgggcgagcc cggcatcttg agtgacatct 1200

gcaaccaagg tctgtcacct gccccaactt tctgcaaccc cacggacaca gcagtcgaga 1260

cccagcctac gtgcaggtca cccccccttg aggtccctga cttgaagaca tcagaggttg 1320

agaaggccag tccctgtcca tcgcctggct cctgtcctcc acccaagagc agcagtgggg 1380

ctccaatgct catcaaagcc aggtccctga gtgtccagca tgtgcccagc tcccaagaag 1440

cagcagaaga tggcatccgc tgccggtctc ggagtatcca gtactgtgtt tcccaagatg 1500

gagctgcctc cctggctgac agcaagccca ccagctcccc gacctccctg aaggcccgtc 1560

catcccagct tcccgtgtca gaccaggcct ctccatgcaa atgcacatgc aaggaaccat 1620

ctcctgtgtc cccagtcact gtgctcaagg cgggaggcac caaagcacct ccccaacacc 1680

tgcccctgtc accagccctg acacgggcag tagaaggcgt ccagtacatt gcagaccacc 1740

tcaaggcaga agacactgac ttctcggtga aggaggactg gaaatacgtg gccatggtca 1800

ttgaccgaat cttcctctgg atgttcatca ttgtctgcct tctgggcact gtgggactct 1860

tcctgcctcc ctggctggct gcttgctga 1889

<210> 10
<211> 29
<212> DNA
<213> Artificial

<220>
<223> Sense primer Chrna4 S286L

<400>  10
cggtgctgct tcttctcacc gtcttcctg                                          29


<210>  11
<211>  29
<212>  DNA
<213>  Artificial

<220>
<223>  Antisense primer Chrna4 S286L

<400>  11
caggaagacg gtgagaagaa gcagcaccg                                          29


<210>  12
<211>  1890
<212>  DNA
<213>  Rattus norvegicus

<400>  12
atggccaatt cgggcaccgg ggcgccgccg ccgctgctgc tactgccgct gctgctgctc      60

ctagggaccg gcctcttgcc tgctagcagc cacatagaga cccgggccca tgcggaggag     120

cggctcctga agagactctt ctccggttac aacaagtggt ctcggccagt agccaatatc     180

tcagatgtgg tcctcgtccg ctttggcttg tccattgctc agctcattga cgtggacgag     240

aagaaccaga tgatgacaac caacgtgtgg gtgaagcagg agtggcacga ctacaagctg     300

cgctgggacc ctggtgacta cgagaatgtc acctccatcc gcatcccctc tgaactcatc     360

tggaggcctg acatcgtcct ctacaacaat gcggatggag actttgcagt cacccacctg     420

accaaggccc acctgttcta tgacggaagg gtgcagtgga cacccccagc catctataag     480

agctcctgca gcatcgacgt caccttcttc ccctttgacc agcagaactg taccatgaag     540

tttggatcct ggacctacga caaggccaag attgacttag tgagcatgca tagccgtgtg     600

gaccaactgg acttctggga aagtggggag tgggtcatcg tggatgctgt gggcacctac     660

aacaccagga agtacgagtg ctgtgccgag atctatcctg acatcaccta tgccttcatc     720

atccgacggc tgccgctatt ctacaccatc aacctcatca tcccgtgcct gctcatctcc     780

tgtctcaccg tgctggtctt ctatctgcct tcagagtgtg gcgagaaggt cacactgtgc     840

atctcggtgc tgcttcttct caccgtcttc ctgctgctca tcaccgagat catcccgtcc     900

acctcgctgg tcatcccgct catcggcgag tacctcctct tcaccatgat cttcgtcacc     960

ctctccatcg tcatcacggt cttcgtgctc aatgtgcacc accgctcgcc acgcacacac    1020

acgatgcccg cctgggtgcg tagagtcttc ctggacatcg tgcctcgcct cctcttcatg    1080

aagcgcccct ctgtggtcaa agacaactgc cggagactta ttgagtccat gcacaagatg    1140

gccaacgccc cccgcttctg gccagagcct gtgggcgagc ccggcatctt gagtgacatc 1200

tgcaaccaag gtctgtcacc tgccccaact ttctgcaacc ccacggacac agcagtcgag 1260

acccagccta cgtgcaggtc accccccctt gaggtccctg acttgaagac atcagaggtt 1320

gagaaggcca gtccctgtcc atcgcctggc tcctgtcctc cacccaagag cagcagtggg 1380

gctccaatgc tcatcaaagc caggtccctg agtgtccagc atgtgcccag ctcccaagaa 1440

gcagcagaag atggcatccg ctgccggtct cggagtatcc agtactgtgt ttcccaagat 1500

ggagctgcct ccctggctga cagcaagccc accagctccc cgacctccct gaaggcccgt 1560

ccatcccagc ttcccgtgtc agaccaggcc tctccatgca aatgcacatg caaggaacca 1620

tctcctgtgt ccccagtcac tgtgctcaag gcgggaggca ccaaagcacc tccccaacac 1680

ctgcccctgt caccagccct gacacgggca gtagaaggcg tccagtacat tgcagaccac 1740

ctcaaggcag aagacactga cttctcggtg aaggaggact ggaaatacgt ggccatggtc 1800

attgaccgaa tcttcctctg gatgttcatc attgtctgcc ttctgggcac tgtgggactc 1860

ttcctgcctc cctggctggc tgcttgctga 1890

<210> 13
<211> 30
<212> DNA
<213> Artificial

<220>
<223> Sense primer Chrna4 c878-879insGCT

<400> 13
gtcttcctgc tgctgctcat caccgagatc 30

<210> 14
<211> 30
<212> DNA
<213> Artificial

<220>
<223> Antisense primer Chrna4 c.878-879insGCT

<400> 14
gatctcggtg atgagcagca gcaggaagac 30

<210> 15
<211> 1893
<212> DNA
<213> Rattus norvegicus

<400> 15
atggccaatt cgggcaccgg ggcgccgccg ccgctgctgc tactgccgct gctgctgctc 60

ctagggaccg gcctcttgcc tgctagcagc cacatagaga cccgggccca tgcggaggag 120

cggctcctga agagactctt ctccggttac aacaagtggt ctcggccagt agccaatatc 180
tcagatgtgg tcctcgtccg ctttggcttg tccattgctc agctcattga cgtggacgag 240
aagaaccaga tgatgacaac caacgtgtgg gtgaagcagg agtggcacga ctacaagctg 300
cgctgggacc ctggtgacta cgagaatgtc acctccatcc gcatcccctc tgaactcatc 360
tggaggcctg acatcgtcct ctacaacaat gcggatggag actttgcagt cacccacctg 420
accaaggccc acctgttcta tgacggaagg gtgcagtgga cacccccagc catctataag 480
agctcctgca gcatcgacgt caccttcttc ccctttgacc agcagaactg taccatgaag 540
tttggatcct ggacctacga caaggccaag attgacttag tgagcatgca tagccgtgtg 600
gaccaactgg acttctggga aagtggggag tgggtcatcg tggatgctgt gggcacctac 660
aacaccagga agtacgagtg ctgtgccgag atctatcctg acatcaccta tgccttcatc 720
atccgacggc tgccgctatt ctacaccatc aacctcatca tcccgtgcct gctcatctcc 780
tgtctcaccg tgctggtctt ctatctgcct tcagagtgtg gcgagaaggt cacactgtgc 840
atctcggtgc tgctttctct caccgtcttc ctgctgctgc taatcaccga gatcatcccg 900
tccacctcgc tggtcatccc gctcatcggc gagtacctcc tcttcaccat gatcttcgtc 960
accctctcca tcgtcatcac ggtcttcgtg ctcaatgtgc accaccgctc gccacgcaca 1020
cacacgatgc ccgcctgggt gcgtagagtc ttcctggaca tcgtgcctcg cctcctcttc 1080
atgaagcgcc cctctgtggt caaagacaac tgccggagac ttattgagtc catgcacaag 1140
atggccaacg ccccccgctt ctggccagag cctgtgggcg agcccggcat cttgagtgac 1200
atctgcaacc aaggtctgtc acctgcccca actttctgca accccacgga cacagcagtc 1260
gagacccagc ctacgtgcag gtcacccccc cttgaggtcc ctgacttgaa gacatcagag 1320
gttgagaagg ccagtccctg tccatcgcct ggctcctgtc ctccacccaa gagcagcagt 1380
ggggctccaa tgctcatcaa agccaggtcc ctgagtgtcc agcatgtgcc cagctcccaa 1440
gaagcagcag aagatggcat ccgctgccgg tctcggagta tccagtactg tgtttcccaa 1500
gatggagctg cctccctggc tgacagcaag cccaccagct ccccgacctc cctgaaggcc 1560
cgtccatccc agcttcccgt gtcagaccag gcctctccat gcaaatgcac atgcaaggaa 1620
ccatctcctg tgtccccagt cactgtgctc aaggcgggag gcaccaaagc acctccccaa 1680
cacctgcccc tgtcaccagc cctgacacgg gcagtagaag gcgtccagta cattgcagac 1740
cacctcaagg cagaagacac tgacttctcg gtgaaggagg actggaaata cgtggccatg 1800
gtcattgacc gaatcttcct ctggatgttc atcattgtct gccttctggg cactgtggga 1860
ctcttcctgc ctccctggct ggctgcttgc tga 1893

<210>  16
<211>  1893
<212>  DNA
<213>  Rattus norvegicus

<400>  16
atggccaatt cgggcaccgg ggcgccgccg ccgctgctgc tactgccgct gctgctgctc      60

ctagggaccg gcctcttgcc tgctagcagc cacatagaga cccgggccca tgcggaggag     120

cggctcctga agagactctt ctccggttac aacaagtggt ctcggccagt agccaatatc     180

tcagatgtgg tcctcgtccg ctttggcttg tccattgctc agctcattga cgtggacgag     240

aagaaccaga tgatgacaac caacgtgtgg gtgaagcagg agtggcacga ctacaagctg     300

cgctgggacc ctggtgacta cgagaatgtc acctccatcc gcatcccctc tgaactcatc     360

tggaggcctg acatcgtcct ctacaacaat gcggatggag actttgcagt cacccacctg     420

accaaggccc acctgttcta tgacggaagg gtgcagtgga cacccccagc catctataag     480

agctcctgca gcatcgacgt caccttcttc ccctttgacc agcagaactg taccatgaag     540

tttggatcct ggacctacga caaggccaag attgacttag tgagcatgca tagccgtgtg     600

gaccaactgg acttctggga aagtggggag tgggtcatcg tggatgctgt gggcacctac     660

aacaccagga agtacgagtg ctgtgccgag atctatcctg acatcaccta tgccttcatc     720

atccgacggc tgccgctatt ctacaccatc aacctcatca tcccgtgcct gctcatctcc     780

tgtctcaccg tgctggtctt ctatctgcct tcagagtgtg gcgagaaggt cacactgtgc     840

atctcggtgc tgctttctct caccgtcttc ctgctgctct taatcaccga gatcatcccg     900

tccacctcgc tggtcatccc gctcatcggc gagtacctcc tcttcaccat gatcttcgtc     960

accctctcca tcgtcatcac ggtcttcgtg ctcaatgtgc accaccgctc gccacgcaca    1020

cacacgatgc ccgcctgggt gcgtagagtc ttcctggaca tcgtgcctcg cctcctcttc    1080

atgaagcgcc cctctgtggt caaagacaac tgccggagac ttattgagtc catgcacaag    1140

atggccaacg ccccccgctt ctggccagag cctgtgggcg agcccggcat cttgagtgac    1200

atctgcaacc aaggtctgtc acctgcccca actttctgca accccacgga cacagcagtc    1260

gagacccagc ctacgtgcag gtcacccccc cttgaggtcc ctgacttgaa gacatcagag    1320

gttgagaagg ccagtccctg tccatcgcct ggctcctgtc ctccacccaa gagcagcagt    1380

ggggctccaa tgctcatcaa agccaggtcc ctgagtgtcc agcatgtgcc cagctcccaa    1440

gaagcagcag aagatggcat ccgctgccgg tctcggagta tccagtactg tgtttcccaa    1500

gatggagctg cctccctggc tgacagcaag cccaccagct ccccgacctc cctgaaggcc    1560

cgtccatccc agcttcccgt gtcagaccag gcctctccat gcaaatgcac atgcaaggaa    1620

ccatctcctg tgtccccagt cactgtgctc aaggcgggag gcaccaaagc acctccccaa    1680

cacctgcccc tgtcaccagc cctgacacgg gcagtagaag gcgtccagta cattgcagac 1740

cacctcaagg cagaagacac tgacttctcg gtgaaggagg actggaaata cgtggccatg 1800

gtcattgacc gaatcttcct ctggatgttc atcattgtct gccttctggg cactgtggga 1860

ctcttcctgc ctccctggct ggctgcttgc tga 1893

<210> 17
<211> 30
<212> DNA
<213> Artificial

<220>
<223> Sense primer Chrnb2 V287L

<400> 17
ctcatctcca agattatgcc tcccacctcc 30

<210> 18
<211> 30
<212> DNA
<213> Artificial

<220>
<223> Antisense primer Chrnb2 S287L

<400> 18
ggaggtggga ggcataatct tggagatgag 30

<210> 19
<211> 1501
<212> DNA
<213> Rattus norvegicus

<400> 19
ggccgggcac tccaactcaa tggcgctgtt cagcttcagc cttctttggc tgtgctcagg 60

ggttttggga actgacacag aggagcggct agtggagcat ctcttagatc cctcccgcta 120

taacaagctg attcgtccag ctactaacgg ctctgagctg gtgactgtac agctcatggt 180

atcattggct cagctcatta gtgtgcacga gcgggagcag atcatgacca ccaatgtctg 240

gctgacccag gagtgggaag attaccgcct cacatggaag cctgaggact tcgacaatat 300

gaagaaagtc cggctccctt ccaaacacat ctggctccca gatgtggttc tatacaacaa 360

tgctgacggc atgtacgaag tctccttcta ttccaatgct gtggtctcct atgatggcag 420

catcttttgg ctaccacctg ccatctacaa gagtgcatgc aagattgagg tgaagcactt 480

cccatttgac cagcagaatt gcaccatgaa gtttcgctca tggacctacg accgtactga 540

gattgacctg gtgctcaaaa gtgatgtggc cagtctggat gacttcacac ccagcgggga 600

gtgggacatc atcgcactgc caggccgacg caacgagaac ccagacgact ccacctatgt 660

ggacatcacc tatgacttca tcattcgtcg caaaccactc ttctacacta tcaacctcat 720

catcccctgc gtactcatca cctcgctggc catcctggtc ttctacctgc cctcagactg 780

tggtgaaaag atgacacttt gtatttctgt gctgctagca ctcacggtgt tcctgctgct 840

catctccaag attctgcctc ccacctccct cgatgtaccg ctggtgggca agtacctcat 900

gtttaccatg gtgctagtca ccttctccat cgtcaccagc gtgtgtgtgc tcaatgtgca 960

ccaccgctcg cctaccacgc acaccatggc cccctgggtc aaggtggtct tcctggagaa 1020

gctgcccacc ctgctcttcc tgcagcagcc acgccaccgc tgtgcacgtc agcgtctgcg 1080

cttgaggagg cgccagcgag agcgtgaggg cgcaggcgcg cttttcttcc gtgaaggtcc 1140

tgcggctgac ccatgtacct gctttgtcaa ccctgcatca gtgcagggct tggctggggc 1200

tttccgagct gagcccactg cagccggccc ggggcgctct gtggggccat gcagctgtgg 1260

cctccgggaa gcagtggatg gcgtacgctt cattgcggac cacatgcgaa gtgaggatga 1320

tgaccagagt gtgagggagg actggaaata cgttgccatg gtgatcgacc gcctgttcct 1380

gtggatcttt gtctttgtct gtgtctttgg gaccgtcggc atgttcctgc agcctctctt 1440

ccagaactac actgccacta ccttcctcca ccctgaccac tcagctccca gctccaagtg 1500

a 1501

<210> .20
<211> 30
<212> DNA
<213> Artificial

<220>
<223> Sense primer Chrnb2 V287M

<400> 20
ctcatctcca agattctgcc tcccacctcc 30

<210> 21
<211> 30
<212> DNA
<213> Artificial

<220>
<223> Antisense primer Chrnb2 S287M

<400> 21
ggaggtggga ggcagaatct tggagatgag 30

<210> 22
<211> 1502
<212> DNA
<213> Rattus norvegicus

<400>  22
tggccgggca ctccaactca atggcgctgt tcagcttcag ccttctttgg ctgtgctcag      60

gggttttggg aactgacaca gaggagcggc tagtggagca tctcttagat ccctcccgct     120

ataacaagct gattcgtcca gctactaacg gctctgagct ggtgactgta cagctcatgg     180

tatcattggc tcagctcatt agtgtgcacg agcgggagca gatcatgacc accaatgtct     240

ggctgaccca ggagtgggaa gattaccgcc tcacatggaa gcctgaggac ttcgacaata     300

tgaagaaagt ccggctccct tccaaacaca tctggctccc agatgtggtt ctatacaaca     360

atgctgacgg catgtacgaa gtctccttct attccaatgc tgtggtctcc tatgatggca     420

gcatcttttg gctaccacct gccatctaca agagtgcatg caagattgag gtgaagcact     480

tcccatttga ccagcagaat tgcaccatga agtttcgctc atggacctac gaccgtactg     540

agattgacct ggtgctcaaa agtgatgtgg ccagtctgga tgacttcaca cccagcgggg     600

agtgggacat catcgcactg ccaggccgac gcaacgagaa cccagacgac tccacctatg     660

tggacatcac ctatgacttc atcattcgtc gcaaaccact cttctacact atcaacctca     720

tcatcccctg cgtactcatc acctcgctgg ccatcctggt cttctacctg ccctcagact     780

gtggtgaaaa gatgacactt tgtatttctg tgctgctagc actcacggtg ttcctgctgc     840

tcatctccaa gattatgcct cccacctccc tcgatgtacc gctggtgggc aagtacctca     900

tgtttaccat ggtgctagtc accttctcca tcgtcaccag cgtgtgtgtg ctcaatgtgc     960

accaccgctc gcctaccacg cacaccatgg ccccctgggt caaggtggtc ttcctggaga    1020

agctgcccac cctgctcttc ctgcagcagc cacgccaccg ctgtgcacgt cagcgtctgc    1080

gcttgaggag gcgccagcga gagcgtgagg gcgcaggcgc gcttttcttc cgtgaaggtc    1140

ctgcggctga cccatgtacc tgctttgtca accctgcatc agtgcagggc ttggctgggg    1200

ctttccgagc tgagcccact gcagccggcc cggggcgctc tgtggggcca tgcagctgtg    1260

gcctccggga agcagtggat ggcgtacgct tcattgcgga ccacatgcga agtgaggatg    1320

atgaccagag tgtgagggag gactggaaat acgttgccat ggtgatcgac cgcctgttcc    1380

tgtggatctt tgtctttgtc tgtgtctttg ggaccgtcgg catgttcctg cagcctctct    1440

tccagaacta cactgccact accttcctcc accctgacca ctcagctccc agctccaagt    1500

ga                                                                   1502

<210>  23
<211>  118
<212>  DNA
<213>  Artificial

<220>
<223>  Probe Chrna4

<400>  23
gggctcacgc cgaagaacgc ctgctcaaaa ggctgttttc tggctataat aaatggtccc      60

gccccgtggc taacatttcc gacgtcgtgc tggtgcggtt cggattatct atcgctca      118

<210>  24
<211>  62
<212>  DNA
<213>  Artificial

<220>
<223>  Probe Chrnb2

<400>  24
aaccccgcct ccgtccaagg actcgccggc gcctttaggg ccgaacctac cgccgctggc      60

cc                                                                    62

<210>  25
<211>  1890
<212>  DNA
<213>  Rattus norvegicus

<400>  25
atggccaatt cgggcaccgg ggcgccgccg ccgctgctgc tactgccgct gctgctgctc      60

ctagggaccg gcctcttgcc tgctagcagc cacatagaga cccgggctca cgccgaagaa     120

cgcctgctca aaaggctgtt ttctggctat aataaatggt cccgccccgt ggctaacatt     180

tccgacgtcg tgctggtgcg gttcggatta tctatcgctc agctcattga cgtggacgag     240

aagaaccaga tgatgacaac caacgtgtgg gtgaagcagg agtggcacga ctacaagctg     300

cgctgggacc ctggtgacta cgagaatgtc acctccatcc gcatcccctc tgaactcatc     360

tggaggcctg acatcgtcct ctacaacaat gcggatggag actttgcagt cacccacctg     420

accaaggccc acctgttcta tgacggaagg gtgcagtgga cacccccagc catctataag     480

agctcctgca gcatcgacgt caccttcttc ccctttgacc agcagaactg taccatgaag     540

tttggatcct ggacctacga caaggccaag attgacttag tgagcatgca tagccgtgtg     600

gaccaactgg acttctggga aagtggggag tgggtcatcg tggatgctgt gggcacctac     660

aacaccagga agtacgagtg ctgtgccgag atctatcctg acatcaccta tgccttcatc     720

atccgacggc tgccgctatt ctacaccatc aacctcatca tcccgtgcct gctcatctcc     780

tgtctcaccg tgctggtctt ctatctgcct tcagagtgtg gcgagaaggt cacactgtgc     840

atcttcgtgc tgctttctct caccgtcttc ctgctgctca tcaccgagat catcccgtcc     900

acctcgctgg tcatcccgct catcggcgag tacctcctct tcaccatgat cttcgtcacc     960

ctctccatcg tcatcacggt cttcgtgctc aatgtgcacc accgctcgcc acgcacacac    1020

acgatgcccg cctgggtgcg tagagtcttc ctggacatcg tgcctcgcct cctcttcatg 1080

aagcgcccct ctgtggtcaa agacaactgc cggagactta ttgagtccat gcacaagatg 1140

gccaacgccc cccgcttctg gccagagcct gtgggcgagc ccggcatctt gagtgacatc 1200

tgcaaccaag gtctgtcacc tgccccaact ttctgcaacc ccacggacac agcagtcgag 1260

acccagccta cgtgcaggtc accccccctt gaggtccctg acttgaagac atcagaggtt 1320

gagaaggcca gtccctgtcc atcgcctggc tcctgtcctc cacccaagag cagcagtggg 1380

gctccaatgc tcatcaaagc caggtccctg agtgtccagc atgtgcccag ctcccaagaa 1440

gcagcagaag atggcatccg ctgccggtct cggagtatcc agtactgtgt ttcccaagat 1500

ggagctgcct ccctggctga cagcaagccc accagctccc cgacctccct gaaggcccgt 1560

ccatcccagc ttcccgtgtc agaccaggcc tctccatgca aatgcacatg caaggaacca 1620

tctcctgtgt ccccagtcac tgtgctcaag gcgggaggca ccaaagcacc tccccaacac 1680

ctgcccctgt caccagccct gacacgggca gtagaaggcg tccagtacat tgcagaccac 1740

ctcaaggcag aagacactga cttctcggtg aaggaggact ggaaatacgt ggccatggtc 1800

attgaccgaa tcttcctctg gatgttcatc attgtctgcc ttctgggcac tgtgggactc 1860

ttcctgcctc cctggctggc tgcttgctga 1890

<210> 26
<211> 1890
<212> DNA
<213> Rattus norvegicus

<400> 26
atggccaatt cgggcaccgg ggcgccgccg ccgctgctgc tactgccgct gctgctgctc 60

ctagggaccg gcctcttgcc tgctagcagc cacatagaga cccgggctca cgccgaagaa 120

cgcctgctca aaaggctgtt ttctggctat aataaatggt cccgccccgt ggctaacatt 180

tccgacgtcg tgctggtgcg gttcggatta tctatcgctc agctcattga cgtggacgag 240

aagaaccaga tgatgacaac caacgtgtgg gtgaagcagg agtggcacga ctacaagctg 300

cgctgggacc ctggtgacta cgagaatgtc acctccatcc gcatcccctc tgaactcatc 360

tggaggcctg acatcgtcct ctacaacaat gcggatggag actttgcagt cacccacctg 420

accaaggccc acctgttcta tgacggaagg gtgcagtgga cacccccagc catctataag 480

agctcctgca gcatcgacgt caccttcttc ccctttgacc agcagaactg taccatgaag 540

tttggatcct ggacctacga caaggccaag attgacttag tgagcatgca tagccgtgtg 600

gaccaactgg acttctggga aagtggggag tgggtcatcg tggatgctgt gggcacctac 660

aacaccagga agtacgagtg ctgtgccgag atctatcctg acatcaccta tgccttcatc 720

atccgacggc tgccgctatt ctacaccatc aacctcatca tcccgtgcct gctcatctcc 780

tgtctcaccg tgctggtctt ctatctgcct tcagagtgtg gcgagaaggt cacactgtgc 840

atctcggtgc tgcttcttct caccgtcttc ctgctgctca tcaccgagat catcccgtcc 900

acctcgctgg tcatcccgct catcggcgag tacctcctct tcaccatgat cttcgtcacc 960

ctctccatcg tcatcacggt cttcgtgctc aatgtgcacc accgctcgcc acgcacacac 1020

acgatgcccg cctgggtgcg tagagtcttc ctggacatcg tgcctcgcct cctcttcatg 1080

aagcgcccct ctgtggtcaa agacaactgc cggagactta ttgagtccat gcacaagatg 1140

gccaacgccc cccgcttctg gccagagcct gtgggcgagc ccggcatctt gagtgacatc 1200

tgcaaccaag gtctgtcacc tgccccaact ttctgcaacc ccacggacac agcagtcgag 1260

acccagccta cgtgcaggtc accccccctt gaggtccctg acttgaagac atcagaggtt 1320

gagaaggcca gtccctgtcc atcgcctggc tcctgtcctc cacccaagag cagcagtggg 1380

gctccaatgc tcatcaaagc caggtccctg agtgtccagc atgtgcccag ctcccaagaa 1440

gcagcagaag atggcatccg ctgccggtct cggagtatcc agtactgtgt ttcccaagat 1500

ggagctgcct ccctggctga cagcaagccc accagctccc cgacctccct gaaggcccgt 1560

ccatcccagc ttcccgtgtc agaccaggcc tctccatgca aatgcacatg caaggaacca 1620

tctcctgtgt ccccagtcac tgtgctcaag gcgggaggca ccaaagcacc tccccaacac 1680

ctgcccctgt caccagccct gacacgggca gtagaaggcg tccagtacat tgcagaccac 1740

ctcaaggcag aagacactga cttctcggtg aaggaggact ggaaatacgt ggccatggtc 1800

attgaccgaa tcttcctctg gatgttcatc attgtctgcc ttctgggcac tgtgggactc 1860

ttcctgcctc cctggctggc tgcttgctga 1890

<210> 27
<211> 1893
<212> DNA
<213> Rattus norvegicus

<400> 27
atggccaatt cgggcaccgg ggcgccgccg ccgctgctgc tactgccgct gctgctgctc 60

ctagggaccg gcctcttgcc tgctagcagc cacatagaga cccgggctca cgccgaagaa 120

cgcctgctca aaaggctgtt ttctggctat aataaatggt cccgccccgt ggctaacatt 180

tccgacgtcg tgctggtgcg gttcggatta tctatcgctc agctcattga cgtggacgag 240

aagaaccaga tgatgacaac caacgtgtgg gtgaagcagg agtggcacga ctacaagctg 300

cgctgggacc ctggtgacta cgagaatgtc acctccatcc gcatcccctc tgaactcatc 360

tggaggcctg acatcgtcct ctacaacaat gcggatggag actttgcagt cacccacctg 420

accaaggccc acctgttcta tgacggaagg gtgcagtgga cacccccagc catctataag 480

agctcctgca gcatcgacgt caccttcttc ccctttgacc agcagaactg taccatgaag 540

tttggatcct ggacctacga caaggccaag attgacttag tgagcatgca tagccgtgtg 600

gaccaactgg acttctggga aagtggggag tgggtcatcg tggatgctgt gggcacctac 660

aacaccagga agtacgagtg ctgtgccgag atctatcctg acatcaccta tgccttcatc 720

atccgacggc tgccgctatt ctacaccatc aacctcatca tcccgtgcct gctcatctcc 780

tgtctcaccg tgctggtctt ctatctgcct tcagagtgtg gcgagaaggt cacactgtgc 840

atctcggtgc tgctttctct caccgtcttc ctgctgctgc taatcaccga gatcatcccg 900

tccacctcgc tggtcatccc gctcatcggc gagtacctcc tcttcaccat gatcttcgtc 960

accctctcca tcgtcatcac ggtcttcgtg ctcaatgtgc accaccgctc gccacgcaca 1020

cacacgatgc ccgcctgggt gcgtagagtc ttcctggaca tcgtgcctcg cctcctcttc 1080

atgaagcgcc cctctgtggt caaagacaac tgccggagac ttattgagtc catgcacaag 1140

atggccaacg ccccccgctt ctggccagag cctgtgggcg agcccggcat cttgagtgac 1200

atctgcaacc aaggtctgtc acctgcccca actttctgca accccacgga cacagcagtc 1260

gagacccagc ctacgtgcag gtcacccccc cttgaggtcc ctgacttgaa gacatcagag 1320

gttgagaagg ccagtccctg tccatcgcct ggctcctgtc ctccacccaa gagcagcagt 1380

ggggctccaa tgctcatcaa agccaggtcc ctgagtgtcc agcatgtgcc cagctcccaa 1440

gaagcagcag aagatggcat ccgctgccgg tctcggagta tccagtactg tgtttcccaa 1500

gatggagctg cctccctggc tgacagcaag cccaccagct ccccgacctc cctgaaggcc 1560

cgtccatccc agcttcccgt gtcagaccag gcctctccat gcaaatgcac atgcaaggaa 1620

ccatctcctg tgtccccagt cactgtgctc aaggcgggag gcaccaaagc acctccccaa 1680

cacctgcccc tgtcaccagc cctgacacgg gcagtagaag gcgtccagta cattgcagac 1740

cacctcaagg cagaagacac tgacttctcg gtgaaggagg actggaaata cgtggccatg 1800

gtcattgacc gaatcttcct ctggatgttc atcattgtct gccttctggg cactgtggga 1860

ctcttcctgc ctccctggct ggctgcttgc tga 1893

<210> 28
<211> 1501
<212> DNA
<213> Rattismprvegocis

<400> 28
ggccgggcac tccaactcaa tggcgctgtt cagcttcagc cttctttggc tgtgctcagg 60

ggttttggga actgacacag aggagcggct agtggagcat ctcttagatc cctcccgcta 120

taacaagctg attcgtccag ctactaacgg ctctgagctg gtgactgtac agctcatggt 180

atcattggct cagctcatta gtgtgcacga gcgggagcag atcatgacca ccaatgtctg 240

gctgacccag gagtgggaag attaccgcct cacatggaag cctgaggact tcgacaatat 300

gaagaaagtc cggctccctt ccaaacacat ctggctccca gatgtggttc tatacaacaa 360

tgctgacggc atgtacgaag tctccttcta ttccaatgct gtggtctcct atgatggcag 420

catcttttgg ctaccacctg ccatctacaa gagtgcatgc aagattgagg tgaagcactt 480

cccatttgac cagcagaatt gcaccatgaa gtttcgctca tggacctacg accgtactga 540

gattgacctg gtgctcaaaa gtgatgtggc cagtctggat gacttcacac ccagcgggga 600

gtgggacatc atcgcactgc caggccgacg caacgagaac ccagacgact ccacctatgt 660

ggacatcacc tatgacttca tcattcgtcg caaaccactc ttctacacta tcaacctcat 720

catcccctgc gtactcatca cctcgctggc catcctggtc ttctacctgc cctcagactg 780

tggtgaaaag atgacacttt gtatttctgt gctgctagca ctcacggtgt tcctgctgct 840

catctccaag attctgcctc ccacctccct cgatgtaccg ctggtgggca agtacctcat 900

gtttaccatg gtgctagtca ccttctccat cgtcaccagc gtgtgtgtgc tcaatgtgca 960

ccaccgctcg cctaccacgc acaccatggc cccctgggtc aaggtggtct tcctggagaa 1020

gctgcccacc ctgctcttcc tgcagcagcc acgccaccgc tgtgcacgtc agcgtctgcg 1080

cttgaggagg cgccagcgag agcgtgaggg cgcaggcgcg cttttcttcc gtgaaggtcc 1140

tgcggctgac ccatgtacct gctttgtcaa ccccgcctcc gtccaaggac tcgccggcgc 1200

ctttagggcc gaacctaccg ccgctggccc ggggcgctct gtggggccat gcagctgtgg 1260

cctccgggaa gcagtggatg gcgtacgctt cattgcggac cacatgcgaa gtgaggatga 1320

tgaccagagt gtgagggagg actggaaata cgttgccatg gtgatcgacc gcctgttcct 1380

gtggatcttt gtctttgtct gtgtctttgg gaccgtcggc atgttcctgc agcctctctt 1440

ccagaactac actgccacta ccttcctcca ccctgaccac tcagctccca gctccaagtg 1500

a 1501

<210> 29
<211> 1502
<212> DNA
<213> Rattus norvegicus

<400> 29
tggccgggca ctccaactca atggcgctgt tcagcttcag ccttctttgg ctgtgctcag 60

gggttttggg aactgacaca gaggagcggc tagtggagca tctcttagat ccctcccgct 120

ataacaagct gattcgtcca gctactaacg gctctgagct ggtgactgta cagctcatgg 180

tatcattggc tcagctcatt agtgtgcacg agcgggagca gatcatgacc accaatgtct 240

ggctgaccca ggagtgggaa gattaccgcc tcacatggaa gcctgaggac ttcgacaata 300

tgaagaaagt ccggctccct tccaaacaca tctggctccc agatgtggtt ctatacaaca 360

atgctgacgg catgtacgaa gtctccttct attccaatgc tgtggtctcc tatgatggca 420

gcatcttttg gctaccacct gccatctaca agagtgcatg caagattgag gtgaagcact 480

tcccatttga ccagcagaat tgcaccatga agtttcgctc atggacctac gaccgtactg 540

agattgacct ggtgctcaaa agtgatgtgg ccagtctgga tgacttcaca cccagcgggg 600

agtgggacat catcgcactg ccaggccgac gcaacgagaa cccagacgac tccacctatg 660

tggacatcac ctatgacttc atcattcgtc gcaaaccact cttctacact atcaacctca 720

tcatcccctg cgtactcatc acctcgctgg ccatcctggt cttctacctg ccctcagact 780

gtggtgaaaa gatgacactt tgtatttctg tgctgctagc actcacggtg ttcctgctgc 840

tcatctccaa gattatgcct cccacctccc tcgatgtacc gctggtgggc aagtacctca 900

tgtttaccat ggtgctagtc accttctcca tcgtcaccag cgtgtgtgtg ctcaatgtgc 960

accaccgctc gcctaccacg cacaccatgg ccccctgggt caaggtggtc ttcctggaga 1020

agctgcccac cctgctcttc ctgcagcagc cacgccaccg ctgtgcacgt cagcgtctgc 1080

gcttgaggag gcgccagcga gagcgtgagg gcgcaggcgc gcttttcttc cgtgaaggtc 1140

ctgcggctga cccatgtacc tgctttgtca accccgcctc cgtccaagga ctcgccggcg 1200

cctttagggc cgaacctacc gccgctggcc cggggcgctc tgtggggcca tgcagctgtg 1260

gcctccggga agcagtggat ggcgtacgct tcattgcgga ccacatgcga agtgaggatg 1320

atgaccagag tgtgagggag gactggaaat acgttgccat ggtgatcgac cgcctgttcc 1380

tgtggatctt tgtctttgtc tgtgtctttg ggaccgtcgg catgttcctg cagcctctct 1440

tccagaacta cactgccact accttcctcc accctgacca ctcagctccc agctccaagt 1500

ga 1502

<210> 30
<211> 629
<212> PRT
<213> Rattus norvegicus

<400> 30

Met Ala Asn Ser Gly Thr Gly Ala Pro Pro Pro Leu Leu Leu Leu Pro
1               5                   10                  15

Leu Leu Leu Leu Leu Gly Thr Gly Leu Leu Pro Ala Ser Ser His Ile
            20                  25                  30

Glu Thr Arg Ala His Ala Glu Glu Arg Leu Leu Lys Arg Leu Phe Ser
        35                  40                  45

Gly Tyr Asn Lys Trp Ser Arg Pro Val Ala Asn Ile Ser Asp Val Val
    50                  55                  60

Leu Val Arg Phe Gly Leu Ser Ile Ala Gln Leu Ile Asp Val Asp Glu
65                  70                  75                  80

Lys Asn Gln Met Met Thr Thr Asn Val Trp Val Lys Gln Glu Trp His
                85                  90                  95

Asp Tyr Lys Leu Arg Trp Asp Pro Gly Asp Tyr Glu Asn Val Thr Ser
            100                 105                 110

Ile Arg Ile Pro Ser Glu Leu Ile Trp Arg Pro Asp Ile Val Leu Tyr
        115                 120                 125

Asn Asn Ala Asp Gly Asp Phe Ala Val Thr His Leu Thr Lys Ala His
    130                 135                 140

Leu Phe Tyr Asp Gly Arg Val Gln Trp Thr Pro Pro Ala Ile Tyr Lys
145                 150                 155                 160

Ser Ser Cys Ser Ile Asp Val Thr Phe Phe Pro Phe Asp Gln Gln Asn
                165                 170                 175

Cys Thr Met Lys Phe Gly Ser Trp Thr Tyr Asp Lys Ala Lys Ile Asp
            180                 185                 190

Leu Val Ser Met His Ser Arg Val Asp Gln Leu Asp Phe Trp Glu Ser
        195                 200                 205

Gly Glu Trp Val Ile Val Asp Ala Val Gly Thr Tyr Asn Thr Arg Lys
    210                 215                 220

Tyr Glu Cys Cys Ala Glu Ile Tyr Pro Asp Ile Thr Tyr Ala Phe Ile
225                 230                 235                 240

Ile Arg Arg Leu Pro Leu Phe Tyr Thr Ile Asn Leu Ile Ile Pro Cys
                245                 250                 255

Leu Leu Ile Ser Cys Leu Thr Val Leu Val Phe Tyr Leu Pro Ser Glu
            260                 265                 270

Cys Gly Glu Lys Val Thr Leu Cys Ile Ser Val Leu Leu Ser Leu Thr
        275                 280                 285

Val Phe Leu Leu Leu Ile Thr Glu Ile Ile Pro Ser Thr Ser Leu Val
    290                 295                 300

Ile Pro Leu Ile Gly Glu Tyr Leu Leu Phe Thr Met Ile Phe Val Thr
305                 310                 315                 320

Leu Ser Ile Val Ile Thr Val Phe Val Leu Asn Val His His Arg Ser
                325                 330                 335

Pro Arg Thr His Thr Met Pro Ala Trp Val Arg Arg Val Phe Leu Asp
            340                 345                 350

Ile Val Pro Arg Leu Leu Phe Met Lys Arg Pro Ser Val Val Lys Asp
        355                 360                 365

Asn Cys Arg Arg Leu Ile Glu Ser Met His Lys Met Ala Asn Ala Pro
    370                 375                 380

Arg Phe Trp Pro Glu Pro Val Gly Glu Pro Gly Ile Leu Ser Asp Ile
385                 390                 395                 400

Cys Asn Gln Gly Leu Ser Pro Ala Pro Thr Phe Cys Asn Pro Thr Asp
                405                 410                 415

Thr Ala Val Glu Thr Gln Pro Thr Cys Arg Ser Pro Pro Leu Glu Val
            420                 425                 430

Pro Asp Leu Lys Thr Ser Glu Val Glu Lys Ala Ser Pro Cys Pro Ser
        435                 440                 445

Pro Gly Ser Cys Pro Pro Pro Lys Ser Ser Ser Gly Ala Pro Met Leu
    450                 455                 460

Ile Lys Ala Arg Ser Leu Ser Val Gln His Val Pro Ser Ser Gln Glu
465                 470                 475                 480

Ala Ala Glu Asp Gly Ile Arg Cys Arg Ser Arg Ser Ile Gln Tyr Cys
                485                 490                 495

Val Ser Gln Asp Gly Ala Ala Ser Leu Ala Asp Ser Lys Pro Thr Ser
            500                 505                 510

Ser Pro Thr Ser Leu Lys Ala Arg Pro Ser Gln Leu Pro Val Ser Asp

        515                 520                 525

Gln Ala Ser Pro Cys Lys Cys Thr Cys Lys Glu Pro Ser Pro Val Ser
    530                 535                 540

Pro Val Thr Val Leu Lys Ala Gly Gly Thr Lys Ala Pro Pro Gln His
545                 550                 555                 560

Leu Pro Leu Ser Pro Ala Leu Thr Arg Ala Val Glu Gly Val Gln Tyr
                565                 570                 575

Ile Ala Asp His Leu Lys Ala Glu Asp Thr Asp Phe Ser Val Lys Glu
            580                 585                 590

Asp Trp Lys Tyr Val Ala Met Val Ile Asp Arg Ile Phe Leu Trp Met
        595                 600                 605

Phe Ile Ile Val Cys Leu Leu Gly Thr Val Gly Leu Phe Leu Pro Pro
    610                 615                 620

Trp Leu Ala Ala Cys
625

<210> 31
<211> 500
<212> PRT
<213> Rattus norvegicus

<400> 31

Met Ala Gly His Ser Asn Ser Met Ala Leu Phe Ser Phe Ser Leu Leu
1               5                   10                  15

Trp Leu Cys Ser Gly Val Leu Gly Thr Asp Thr Glu Glu Arg Leu Val
            20                  25                  30

Glu His Leu Leu Asp Pro Ser Arg Tyr Asn Lys Leu Ile Arg Pro Ala
        35                  40                  45

Thr Asn Gly Ser Glu Leu Val Thr Val Gln Leu Met Val Ser Leu Ala
    50                  55                  60

Gln Leu Ile Ser Val His Glu Arg Glu Gln Ile Met Thr Thr Asn Val
65                  70                  75                  80

Trp Leu Thr Gln Glu Trp Glu Asp Tyr Arg Leu Thr Trp Lys Pro Glu
                85                  90                  95

Asp Phe Asp Asn Met Lys Lys Val Arg Leu Pro Ser Lys His Ile Trp
                100                 105                 110

Leu Pro Asp Val Val Leu Tyr Asn Asn Ala Asp Gly Met Tyr Glu Val
        115                 120                 125

Ser Phe Tyr Ser Asn Ala Val Val Ser Tyr Asp Gly Ser Ile Phe Trp
    130                 135                 140

Leu Pro Pro Ala Ile Tyr Lys Ser Ala Cys Lys Ile Glu Val Lys His
145                 150                 155                 160

Phe Pro Phe Asp Gln Gln Asn Cys Thr Met Lys Phe Arg Ser Trp Thr
                165                 170                 175

Tyr Asp Arg Thr Glu Ile Asp Leu Val Leu Lys Ser Asp Val Ala Ser
            180                 185                 190

Leu Asp Asp Phe Thr Pro Ser Gly Glu Trp Asp Ile Ile Ala Leu Pro
        195                 200                 205

Gly Arg Arg Asn Glu Asn Pro Asp Asp Ser Thr Tyr Val Asp Ile Thr
    210                 215                 220

Tyr Asp Phe Ile Ile Arg Arg Lys Pro Leu Phe Tyr Thr Ile Asn Leu
225                 230                 235                 240

Ile Ile Pro Cys Val Leu Ile Thr Ser Leu Ala Ile Leu Val Phe Tyr
                245                 250                 255

Leu Pro Ser Asp Cys Gly Glu Lys Met Thr Leu Cys Ile Ser Val Leu
            260                 265                 270

Leu Ala Leu Thr Val Phe Leu Leu Leu Ile Ser Lys Ile Val Pro Pro
        275                 280                 285

Thr Ser Leu Asp Val Pro Leu Val Gly Lys Tyr Leu Met Phe Thr Met
    290                 295                 300

Val Leu Val Thr Phe Ser Ile Val Thr Ser Val Cys Val Leu Asn Val
305                 310                 315                 320

His His Arg Ser Pro Thr Thr His Thr Met Ala Pro Trp Val Lys Val
                325                 330                 335

Val Phe Leu Glu Lys Leu Pro Thr Leu Leu Phe Leu Gln Gln Pro Arg
            340                 345                 350

His Arg Cys Ala Arg Gln Arg Leu Arg Leu Arg Arg Arg Gln Arg Glu
        355                 360                 365

Arg Glu Gly Ala Gly Ala Leu Phe Phe Arg Glu Gly Pro Ala Ala Asp
    370                 375                 380

Pro Cys Thr Cys Phe Val Asn Pro Ala Ser Val Gln Gly Leu Ala Gly
385                 390                 395                 400

Ala Phe Arg Ala Glu Pro Thr Ala Ala Gly Pro Gly Arg Ser Val Gly
                405                 410                 415

Pro Cys Ser Cys Gly Leu Arg Glu Ala Val Asp Gly Val Arg Phe Ile
            420                 425                 430

Ala Asp His Met Arg Ser Glu Asp Asp Asp Gln Ser Val Arg Glu Asp
        435                 440                 445

Trp Lys Tyr Val Ala Met Val Ile Asp Arg Leu Phe Leu Trp Ile Phe
    450                 455                 460

Val Phe Val Cys Val Phe Gly Thr Val Gly Met Phe Leu Gln Pro Leu
465                 470                 475                 480

Phe Gln Asn Tyr Thr Ala Thr Thr Phe Leu His Pro Asp His Ser Ala
                485                 490                 495

Pro Ser Ser Lys
            500